Europäisches Patentamt

(19) European Patent Office

Office européen des brevets

(11) Publication number: **0 048 451**

**A1**

(12) **EUROPEAN PATENT APPLICATION**

(21) Application number: **81107360.0**

(22) Date of filing: **17.09.81**

(51) Int. Cl.³: **A 61 N 1/42**

(30) Priority: **24.09.80 US 190222**

(43) Date of publication of application:
**31.03.82 Bulletin 82/13**

(84) Designated Contracting States:
**AT BE CH DE FR GB IT LI LU NL SE**

(71) Applicant: **Drolet, Roland A.**
**1150 de la Toison d'Or**
**Les Saules Quebec(CA)**

(72) Inventor: **Drolet, Roland A.**
**1150 de la Toison D'Or**
**Les Saules Quebec(CA)**

(72) Inventor: **Charland, Gaétan**
**60 Place Orléans, Apt. 7**
**Beauport Quebec(CA)**

(74) Representative: **Radt, Finkener, Ernesti Patentanwälte**
**Heinrich-König-strasse 119**
**D-4630 Bochum 1(DE)**

(54) **Electro-magnetic therapeutic system and method.**

㊳ An electromagnetic low-frequency therapeutic system and method. The system comprises a magnetization coil for creating an electro-magnetic field. Securing means is provided to secure the coil in a predetermined fixed position. A generator feeds the coil with predetermined treatment signals to obtain a desired magnetic field characteristic. Control circuit means is provided for selecting desired characteristics of the treatment signals. The control circuit means has a circuit control means for controlling the peak intensity of the desired magnetic field. The control circuit further is provided with frequency control means to select the interruption frequency of the treatment signals to obtain a selected type of a plurality of treatment signals. Adjustment means is also provided to preset the duration time of the treatment signals and the electro-magnetic field. The desired magnetic field characteristic is predetermined from a magnetic field pattern chart representative of the parameters of the field of the magnetization coil in the surrounding environment of the coil.

EP 0 048 451 A1

./...

Fig. 1

(A) GENERATOR (OR CONTROL UNIT)

(B)

(C)

(D)

(E)

The present invention relates to an improved low frequency electromagnetic therapeutic system and method. In every country of the world, there is approximately 1 person in 10 suffering from some form of arthritic diseases and bone fractures leading to arthritic diseases, and drugs presently used to treat these conditions often cause harmful side effects with prolonged use. These considerations and our knowledge of the generalized therapeutic value of Low-Frequency Electro-Magnetic (LF-EM) fields led us to develop a LF-EM therapy system, which would be non-invasive, not costly to the patient, would leave lasting benefits without the continual use of anti-inflammatory drugs, and which would be used by non-specialists. This form of therapy would be available at short notice to all suffering patients.

This was made possible with LF-EM fields (pulsatile and sinusoidal), after several years of research which led to the successful development of "RHUMART"-THERAPY. (The word "RHUMART" is a Registered Trade Mark for the present invention).

(b) Description of Prior Art

The following references are referred to hereinafter.

State of the art in rheumatology:

The underlying mechanism causing arthritis and related diseases are far from being understood. The basic treatment is still ASPIRIN (Registered Trademark), steroids and other anti-inflammatory drugs, none of which produce a lasting or permanent cure, and all

- 1 -

[1] Brighton, C.T. (M.D., Ph. D., and Guest Editor), 1977, Clin. Orthop. Rel. Res., No. 124, May 1977.

[2] Bassett, C.A.L., 1978, Skeletal Effects of Pulsing Electromagnetic Fields, AAMI, 13th Annual Meeting, March 28-April 1st, 1978, Washington D.C.

[3] Adey, W.R., and S.M. Banvin, 1976, Brain Interactions with Weak Electric and Magnetic Fields, Neurosciences Res. Prog. Bull., Vol. 15, No. 1, 1-129.

[4] Gavalas, R.J. Walter, D.O. Hamer, J., and W.R. Adey, 1970, Effect of low-level, low-frequency electric fields on EEG and behavior in Macaca nemestrina, Brain Res., Vol 18: 491-501.

[5] Wever, R., 1968, Einfluss schwacher elektromagnetischer Felder auf die circadiane Periodik des Menschen, Naturwissenschaften, 1: 29-33.

[6] Baker, M.A., and J.N. Hayward, 1967, Carotid rete and brain temperature of cat, Nature (London), 216: 139-141.

[7] Hollander, J.L. Horvath, S.M. 1949, Influence of physical therapy procedures on intra-articular temperature of normal and arthritic subjects, Am. J. Med. Sci. 218: 543-548.

[8] Fiebel, A., and F. Avital, 1976, Deep Heating of Joints: A Reconsideration, Arch. Phys. Med. Rehabil., Vol. 57, Nov. 1976.

[9] Licht, S.H., 1965, Therapeutic Heat and Cold, Ed. 2, New Haven, E. Licht.

[10] Lehmann, J.F., Warren, C.G., and Scham, S.M., 1974, Clin. Orthop., 99: 207-245.

[11] Harris, E.D. Jr., and McCrosbery, P.A., 1974, Influence of temperature and fibril stability on degradation of cartilage collagen by rheumatoid synovial collagenase, N. Engl. J. Med., 290: 1-6.

[12] Harris, E.D., 1974, Rheumatoid Arthritis, New York, MEDCOM Press, pp. 25, 87, 50-51.

[13] Drolet, R. and Kunov, H., 1969, Physical Interpretation of Biological Impedances with Applications to Electro-Stimulation, 2nd CMBES Conference, Halifax.

[14] Tabili, M.A., Drolet, R. et al., 1970, A. Model for Studying the Electrical Stimulation of the Urinary Bladder of Dogs, British J. Urol., Vol. 42, 56-65.

of which produce undesirable/harmful side effects after prolonged use. The state of the art in rheumatology is reflected by the following quotation from the recent book "The Recognition of Anti-Rheumatic Drugs" edited by Dr. D.C. Dumonde of the Matilda and Terence Kennedy Institute of Rheumatology, London, England:

"Rheumatoid arthritis and allied inflammatory diseases represent a major challenge to medical practice and pharmaceutical enterprise. -- On the one hand, we now admit that the term "rheuma-'tism" is without aetiological or pathogenetic significance and the recognition of inflammatory rheumatic disease relies largely upon our ability to assemble deviations from normality into certain patterns which may partly fullfil our national classification criteria. -- On the other hand, we also admit that current treatment and control of inflammatory rheumatic disease, though increasingly more powerful, represents a "halfway" stage of achievement."

Concerning the underlying mechanism, the same author states:

"There is a growing view that rheumatoid arthritis and allied diseases arise from subtle defects in the regulation of immunological and inflammatory responses upon which both genetic and environmental factors may find expression."

-Electromagnetic hypothesis :

Our consistently good clinical results in numerous arthritic diseases using LF-EM fields and that of others cited hereinafter led us to formulate the following hypothesis: many rheumatic diseases may be associated with local electromagnetic pertubations, and it is therefore logical that these diseases are influenced by low frequency electromagnetic fields. This hypothesis is compatible with Dr. Dumonde's statement cited above, to the effect that <u>environmental factors</u> may find expression in the subtle defects causing rheumatoid arthritis and allied diseases.

-<u>Low-Frequency Electromagnetic (LF-EM) Therapy</u>:

Numerous beneficial biomedical applications of Low-Frequency Electromagnetic therapy (sometimes identified as magnetotherapy) have been reported in the scientific field and the Patent literature as revealed by the literature cited hereinafter, starting as early as 1904 with U.S. Patent No. 90,732 and numerous other publications.

Solov'eva (1975), Instrumentation and Application of Low-Frequency Magnetotherapy, Biomed, Eng., Vol. 8, No. 3, defines magnetotherapy as consisting of the application of low-frequency magnetic fields: 50 Hz sinusoidal fields, 50 or 100 Hz pulsating fields, periodically interrupted fields, and sometimes also rotating magnetic fields. It was reported in numerous publications, as for example those of Solov'eva (1975) and Mizushima et al. (1975) Effects of Magnetic Field on Inflammation, Experientia, Vol. 31, 12 , that the effectiveness of magnetotherapy is beyond any doubt in numerous therapeutic applications.

However, the results of applications of magnetotherapy are frequently not uniform mainly because of the different specific characteristics of the instruments used and because of the numerous different ways and/or procedures adopted by the users of a given instrument. It is also recognized that constant magnetic fields have only limited application in medical practice (Solov'eva 1975).

The state of the art of Magnetotherapy, as defined above, is well summarized in Solov'eva's paper (1975). Numerous apparatus and clinical results obtained with them are described in Solov'eva's paper. Two types of instruments are reviewed in his paper: those using electromagnet inductor(s) and those using solenoid inductor(s). According to Solov'eva, in an electromagnet, the field intensity is maximum at the core ends (poles) and rapidly falls off with distance, so that in use the pathological focus should be as close as possible to one of the electromagnet poles. The same author states that the field set up by a solenoid inductor is strongest in its inner, hollow part, so that in application the pathological region of the body should be surrounded by the solenoid windings (Solov'eva, 1975). However, because of the absence of a core, much power is consumed by the solenoid to set up sufficiently strong fields and, the therapeutic application of solenoids is usually accompanied by the release of considerable heat using prior apparatus.

Using prior magnetotherapy apparatus, satisfactory results have been obtained in the treatment of trophic ulcers, slowly healing wounds, burns, postoperative infiltrates, gastric diseases, algesic syndrome associated with traumatic injuries of the peripheral nerves, cardio-vascular diseases with decompensation

- 4 -

effects, bronchial asthma, otorhinolaryngological ailments, diseases of the nervous system, supportive and motorial apparatus, gynecological and skin diseases, impaired functioning of the joints and ligaments, heel bursitis (spurs), traumatic edema, pathological motorial functions in children. Improvements of the general state of the patient, a reduction of itching, improved epithelization of ulcerous surfaces and anesthetic effects have also been observed with magnetotherapy.

Furthermore, physiological and histochemical data indicate a sedative effect of continuous sinusoidal or pulsating fields associated primarily with assimilation processes and a stimulating effect of intermittent fields associated mainly with dissimilation processes (Solov'eva). It is also reported in the literature that sedative modes of treatment are indicated in sympaticotonia and orthosympaticotonia, in asthenic neurosis, neurasthenia, contractures, arthrosis, spondylitis, active rheumatism, and complications following viral hepatitis. Stimulating modes are used in parasympaticotonia, depressive neurosis, bronchial asthmia, and inactive rheumatism. A combination of sedative and stimulating modes is effective in algesic and trophic disorders in the extremities. Gynecological disorders were treated using different operation modes and their combinations.

Prior studies have shown that the direction in which new nerve processes grow can be controlled by weak electric fields which fields may influence regeneration by directing nerves into the region where they influence blastema formation (R. B. Borgens, 1979, Bio-Electricity and Limb Regeneration, Encyclopedia Science, Suppl. 1979, pp. 89-93 (Biology) Grolier Ltd., Toronto, Canada).

Several authors who investigated Low-Frequency Electro-Magnetic Field reported anti-inflammatory effects (Mizushima, 1975), normalization of arterial pressure, diuretic action, normalization of transmembrane electrical potential of living cells, stimulation of osteogenesis and bone repair and maintenance (using specific current pulses (Brighton[1], 1977), alteration of the amount and rate of calcium influx-efflux at the cellular level (Bassett[2], 1978), therapeutic effects on the nervous system without significant heating of tissues (Adey[3], 1973; Gavalas[4], 1970, Wever[5], 1968; Baker[6], 1967), improvement of sleep and pain relief in all kinds of arthritic diseases.

Furthermore, several authors have noted the advantages of magnetotherapy over UHF (Ultra High Frequency) therapy and induction heating (in the book entitled "Biological and Therapeutic Effects of Magnetic Field and Strictly Periodic Vibrations", in Russian, Perm' 1948). One of the reasons for which magnetotherapy give better results than UHF therapy for treating arthritic joints is that deep joint heating associated with UHF fields and diathermy apparatus causes severe side effects and can accelerate cartilage degeneration (i.e., Hollander et al.[7], 1949; Feibel et al.[8], 1976; Licht[9], 1965; Lehmann et al.[10], 1974; Harris et al.[11], 1974; Harris[12], 1974).

The measured effects induced with Low-Frequency Electro-Magnetic (LF-EM) Fields can be explained neither with classical electrophysiology nor with classical neurochemistry. Therefore, new theories were developed to explain the experimental evidence (Adey, 1976). The LF-EM fields not only affect the surface tissues but also permeate bones and the whole organism, and influence all living cells. Our first involvement in Low-Frequency Electromagnetic field applications was in 1969 (Drolet[13] et al., 1969, Talibi[14] et al., 1970) and it was concerned with the evacuation of the bladder of paraplegic patients, at the Institute of Biomedical Electronics and Engineering of the University of Toronto, Canada. We have studied the <u>optimal para-meters</u> for the electromagnetic evacuation of the urinary bladder including electrode characteristics and different electrical current characteristics.

The U.S. and German Patent literature is indicative of the increasing importance of LF-EM field in medical treatment.

It is important to notice that recently three different instruments using electric current won FDA approval in the U.S.A. for the acceleration of bone repair in fractures that remained open for long periods of time. Only one of these instruments is non-invasive. Much of the prior art in this field is described in Vol. 238 of the Annals of the New York Academy of Sciences published October 11, 1974, and entitled "Electrically Mediated Growth Mechanism in Living Systems" (Edited by A. R. Liboff and R. A. Rinaldi).

Patent literature on electromagnetic therapy was published as early as in 1904. Indeed, an electro-magnetic therapeutic apparatus using electromagnets or coils was patented in 1904 in U.S. Pat. No. 90,732. Hence, the principle of using electromagnetic fields for medical treatment has been known for at least eighty (80) years.

Benson (1922) U.S. Pat.No. 1,418,903 described a body receiving electromagnetic coil with means within the electromagnetic coil for generating variable induced currents and applying same to portions of the body under treatment.

Mann (1927) U.S. Pat. No. 1,634,373 described an ELECTRIC THERAPEUTIC DEVICE which produces heat, vibrations and eddy currents for the purpose of treating the human body.

Mc Lean (1972) U.S. Pat. No. 3,658,051 presented an apparatus and method for treating a living thing or part thereof with intermittent and continuous high intensity (higher than 2,000 gauss) magnetic fields using a pulse frequency of about 2 Hz.

Hallgren (1974) U.S. Pat. No. 3,841,305 illustrated a system for external stimulation for a nerve including a coil of wire with a specific flux-concentrating core (preferably having a T-shape), in which the coil is pulsed by a discharging capacitor and specific circuits are disclosed for charging the capacitor and generating discharge pulses of alternate polarity.

Liss et al. (1975) U.S. Pat. No. 3,902,502 described an apparatus for temporarily arresting arthritic pain at local areas of a patient. The apparatus has
conducting leads for carrying the output of the apparatus
to contact ends (electrodes) applied to the skin of
the patient at a localized area. This system uses a
carrier frequency of between 20 k Hz and 1 M Hz which
is amplitude modulated (ON - OFF) at very low frequencies between 10 and 40 Hz. The output of the apparatus
is applied for a short period such as 3 to 4 minutes
to the treated local area.

Paul Jr. (1975) U.S. Pat. No. 3,881,494
discloses an electro-pulse system for providing temporary pain relief to arthritic patients through therapeutic use of an electronic circuit involving a self-
repetitive capacitive discharge technique, and a pair
of electrodes through which current is flown through
the treated area of the patient.

Kraus et al. (1975) U.S. Pat. No. 3,890,953
described an electrical apparatus for promoting the
growth of bone and other body tissues by the application
thereto of a low-frequency alternating magnetic field.
The apparatus consists essentially of a current generator
and one or many field applicators. The improvement
resides in that the applicator means comprises a flat
solenoid coil having an axis about which the coil is
wound and composed of a plurality of parallel and
flexible windings. Each winding has two adjacent
elongate portions and two $180^{\circ}$ coil bends joining the
elongate portions together. The frequency of the A.C.
current generator is below 150 Hz in the apparatus.

Kraus described different coil structures for electromagnetic therapy: oval or elliptical cylinder and inclined cylinder (U.S. Pat. No. 4,066,065, 1978; and corresponding German Pat. No. 2432 493, 1976); prior to the two Patents just cited above, W. Kraus (1974) was author of the German Pat. No. 2,314,573 in which an electromagnetic therapeutic apparatus for the acceleration of bone growth (according to Figures) is described. His apparatus is composed of at least one electric field applicator (two electrodes) and one magnetic field applicator (coil, single and multiple); and the frequency of the A.C. magnetic field is between 1 and 100 Hz with an intensity of between 20 and 200 gauss, and the intensity of the electric field applied being between 0.1 V/cm and 1 V/cm. The shape of certain coils used in the later apparatus look similar to that of the coils described by the same author in the above cited U.S. Patent No. 3,890,953.

R. Buschky (German Pat. No. 2,517,896, 1976 and No. 2,533,244, 1977) describes two different magnetic field applicators (both cylindrical in shape) which are either adapted in a flexible way on a carriage or wall mounted with movable parts. The mechanical structures described in these two patents look bulky and heavy.

M. Buschky (1977) German Pat. No. 2,553,197 described an impulse field generator generating Low-Frequency currents in the range of 1 Hz to 1 k Hz.

Maass (1977) U.S. Pat. No. 4,056,097 describes a contactless stimulus transducer which induces a stimulus current into a biological specimen by means of a changing magnetic field which is produced by an electric field winding on a ferro-magnetic core.

Lastly, Goldman et al. (1978) U.S. Pat. No. 4,095,588 patented a method of cleansing a vascular system comprising arranging a plurality of electromagnetic coils to be separate from each other about an axis in the form of an annular electromagnetic means. The coils are elongated in a direction perpendicular to the magnetic axis of the coils for encircling with a magnetic field controllable by a variable frequency and amplitude to propel red blood cells radially while rotating about a vascular axis so as to loosen and clear away vascular accumulations tending to block the vascular system.

OBJECTS AND SUMMARY OF THIS INVENTION

In contrast to the prior inventions, while also relating to specific biomedical applications of alternating and/or pulsating magnetic fields, this invention is concerned with an original compact, portable, mobile and modular Low-Frequency Electromagnetic (LF-EM) Therapy System having mechanically and electronically compatible and interchangeable subsystems or units, and including two LF field generators, one pulsating and one sinusoidal generator, both of which can generate continuous or periodically interrupted treatment signals of specific characteristics.

Three special treatment units or field applicators, herein referred to as MINI, MOYI and MAXI; and four different types of flexible and/or adaptable mechanical supports (three of which contain one or two adjustable-pressure disc-breaks), for manipulating the treatment applicators in any desired direction in space; a simple support for MINI field applicator, floor and/or wall supports for the three field applicators (MINI, MOYI, and MAXI), and a 4-wheel carriage support for increased mobility of any of the three field applicators (this mobile carriage can be used for treating patients in standard hospital beds or otherwise.)

The present improved Electromagnetic Therapy System has a wide range of biomedical applications because of the following features:
- wide range of controllable treatment characteristics,
- well localized treatment in the affected area of the patient;
- non-thermal-treatment;
- painless treatment;
- treatment without mechanical vibration;
- treatment applied without metallic electrodes;
- treatment by electromagnetic induction of low frequency currents of desired characteristics within the treated region of the body;
- specific magnetization-demagnetization feature of the sinusoidal generator;
- specific time pattern of treatment;
- modular structure (interchangeable generators, Treatment Units and mechanical supports);
- low weight;
- portability;

- mechanical flexibility;

- wide range of controllable electromagnetic field parameters (field intensity, direction, frequency and spatial gradient) generated by one sinusoidal and/or one pulsating field generator;

- Critical-Damping-Design for the pulse-shaping-circuit of the pulsating generator, allowing for a great improvement of the field intensity and effectiveness for a given weight of field applicator and/or generator.

This system can generate numerous desirable, controllable and useful Low-Frequency Electro-Magnetic (LF-EM) treatment characteristics.

More specifically, as further features of the present invention, the following parameters of the LF-EM field can be selected or predetermined by this system:

- Peak amplitude of sinusoidal and pulsating fields. With the sinusoidal generator the peak amplitude can be programmed to remain constant, at a preselectable level (0 to 100 gauss in ten steps when using MAXI, 0 to 300 gauss in ten steps when using MOYI, and 0 to 900 gauss in ten steps when using MINI) for a preselectable time MAG TIME (0.5, 1.0, 1.5, 2.0, 2.5, 3.0, 5, 10, 15, 20, 25 and 30 minutes) and then to automatically drop to zero amplitude, to decrease linearly or quasi exponentially to zero amplitude, in a preselectable time DEMAG TIME (0.5, 1.0, 1.5, 2.0, 2.5, 3.0, 5, 10, 15, 20, 25 and 30 minutes).

- <u>Orientation and/or direction</u> with respect to patient or part thereof, of sinusoidal and pulsating fields in space using the four (4) different types of modular mechanical support and/or carriage described hereinafter. The Orientation of the field can be in any desired direction.

- <u>The frequency</u>, in cycles per second, meaning the repetition frequency of pulse bundles (with the pulsatile generator) or the repetition frequency of sinusoidal wave bundles (with the sinusoidal generator); can be selected as 60 Hz, 30 Hz, 15 Hz, 7.5 Hz, 3.75 Hz and 1.875 Hz when 60 Hz power is supplied to the generator; and 50, 25, 12.5, 6.25, 3.125, and 1.56 Hz when a 50 Hz power is supplied to the generator, (1, 2, 4, 8 or 16 elementary sine wave cycle(s) or current pulse(s) are generated in bundles and each bundle is followed by an interruption time (zero field) equal to the time of the said bundle before the origination of a new treating wave bundle, and so on.

- <u>Time of treatment</u> is preselectable for both sinusoidal and pulsatile generators as indicated in the above subparagraph entitled "peak amplitude of sinusoidal and pulsating fields".

- <u>The spatial field gradient</u> in the patient and/or treatment region can be preset by choosing the proper Treatment Unit (MINI, MOYI or MAXI); and placing it at a proper distance and/or orientation with respect to the patient and/or treatment region, with the guidance of the so-called "FIELD PATTERN CHART" corresponding to each treatment unit as described hereinafter.

- <u>The volume of the treated region of the patient</u>, for a given range of field (from 20 to 50 gauss for example), can be preset also by choosing the proper Treatment Unit (MINI, MOYI, or MAXI), and placing it at a proper distance and/or orientation with respect to the patient and/or treatment region, with the guidance of the "FIELD PATTERN CHART" corresponding to each treatment unit as described hereinafter.

An important original feature of the pulse shaping circuit (of the pulsatile generator) is that the electronic components of this circuit are chosen so as to obtain a critical or "nearly critical" damping of the current pulse flowing through the Treatment Unit. This "Critical Damping Condition" is defined by the simultaneous solution of the three following equatÓns:

$$C = \frac{4L}{R_L^2} \; ; \quad t_m = \frac{2L}{R_L} \; ; \quad \text{and } i_m = -(\frac{R_L}{2L}) \frac{C \, V_o}{e}$$

where C = capacitance of capacitor being discharged into the treatment coil through a damping resistor $R_{Damping}$;

L = inductance of the treatment coil;

$R_L$ = total series resistance in the discharging circuit of the capacitor; $R_L$ is equal to the sum of the damping resistor $R_{Damping}$ and the D.C. electrical resistance $r_L$ of the treatment coil winding;

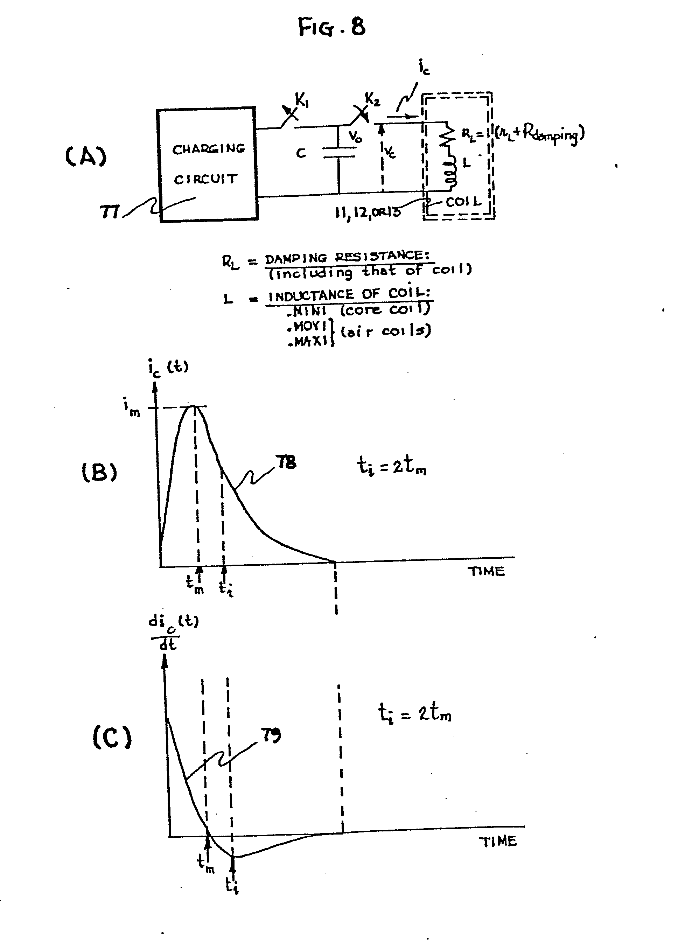

$t_m$ = time lapse between beginning of the current pulse and the time corresponding to the maximum current $i_m$, as illustrated in Figure 8 herein;

$t_i$ = period of time from the beginning of the current pulse to the time where the second derivative of the current in this pulse is equal to zero. (We have shown that $t_i = t_m/2$, see Figure 8 herein);

$i_m$ = maximum current flowing through the treatment coil;

$V_o$ = initial voltage across the capacitor C, before discharging it into the treatment coil;

$e$ = 2.7182818...., being the limit approached by the expression $(1 + \frac{1}{n})^n$ as n approaches infinity, also called the "base" of natural or neperian logarithm;

and it is intended that any of the components of this R, L, C circuit can be changed so as to cause the peak amplitude of the said current pulse to decrease by not more than 50% of that resulting from the "Critical Damping Conditions" defined above, without departing from the spirit and scope of the present invention. It can be shown that: $i = -(R_L/2L)^2\, CV_o te^{-(R_L/2L)t}$ where t is the time and i the current flowing through the coil, for the critical damping design.

We have "optimized" the present invention in order to increase its usefulness for the following biomedical applications: _in Orthopedics_, for the acceleration of bone growth and repair mechanism and for accelerating the recession of inflammation often occuring after bone fractures; _in Rheumatology_ for causing the recession

- 16 -

of inflammation, the relief of pain, the normalization 0048451

of transmembrane potentials of affected cells, the normalization of the amount and rate of calcium influx-efflux at the cellular level, beneficial long lasting effects on the nervous system, improved sleep and improved general condition of patients suffering from most kinds of arthritic diseases; in Chiropractic for treating various traumatic skeletal and muscular injuries without causing pain to the patient; for treating various Geriatric and Neurological disorders like brachial neuralgia, intercostal neuralgia, lumbalgia, head neuralgia, disturbed sleep, nervous depression and certain migraines; in Physiatry centers for sport injuries; and in Veterinarian clinics for treating severe skeletal and muscular injuries to valuable livestock; for the treatment of trophic ulcers, slowly healing wounds, burns, post-operative infiltrates, gastric diseases, algegic syndrome associated with traumatic injuries, diseases with decompensation effects, bronchial asthma, otorhinolaryngological ailments, diseases of the nervous system, gynecological and skin diseases, impaired functioning of the joints and ligaments, heel bursitis (spurs), traumatic edema, pathological motorial functions in children, improving the general state of the patient, improving epithelization of ulcerous surfaces, inducing anesthetic effects; for inducing a sedative effect associated primarily with assimilation processes and a stimulating effect associated mainly with dissimination processes; for inducing a sedative effect in sympaticotonia and orthosympaticotonia, in astenic neurosis, neurasthenia, contractures, arthrosis, spondylitis, active rheumatism; for inducing a stimulating effect in parasympaticotonia, depressive neurosis, bronchial asthmia and inactive rheumatism.

According to a broad aspect of the present invention there is provided an electro-magnetic low-frequency therapeutic system comprising a magnetization coil for creating an electro-magnetic field, means to secure said coil in a predetermined fixed position, generator means for feeding said coil with predetermined treatment signals to obtain a desired magnetic field characteristic, control circuit means for selecting desired characteristics of said treatment signals, said control circuit means having (i) circuit control means for controlling the peak intensity of said desired magnetic field, (ii) frequency control means to select the interruption frequency of said treatment signals to obtain a selected type of a plurality of therapeutic signals, (iii) adjustment means to preset the duration time of said treatment signals and said electro-magnetic field, said desired magnetic field characteristic being predetermined from a magnetic field pattern chart representative of the parameters of the field of said magnetization coil in the surrounding environment of said coil whereby to obtain a desired range of intensity of the field and a desired orientation thereof relative to a position of the coil.

According to a still further broad aspect of the present invention there is provided a method of obtaining a desired electro-magnetic field at points in space in close surrounding environment of a magnetization coil comprising the steps of: (i) providing a field pattern chart of said coil to display the orientation of iso-magnetic field lines of said coil to define the pattern of the intensity and to display the direction of said magnetic field in space, said field lines being relative to a scale with respect to said coil; (ii) selecting an area in said field pattern chart having a desired field intensity range and field directions; (iii) orienting said coil with respect to a desired location in space to position said selected area thereon; (iv) generating controlled treatment signals to said coil to obtain said desired field intensity range and to select a time/frequency/amplitude/modulation of said field intensity; and (v) applying said treatment signals for generating said magnetic field for a predetermined period of time.

BRIEF DESCRIPTION OF DRAWINGS

A preferred embodiment of the present invention will now be described with reference to the accompanying drawings in which:

FIGURES 1A to 1E are perspective illustrations of four possible configurations of the system of this invention:

FIGURE 2A is a perspective exploded view of the components of the articulated joint used in different mechanical supports of the system of Figures 1B, 1D and 1E;

FIGURE 2B is a perspective view of the mechanical part which holds the horizontal and vertical rods together on the 4-wheel carriage illustrated in Fig. 1E;

FIGURE 2C is a fragmented perspective view of the mechanical holding structure used to hold the vertical rod removably fixed on to the 4-wheel carriage as shown in Figure 1E;

FIGURE 3 is a block diagram illustrating possible combinations of the field generators, treatment applicators and mechanical supports of the system;

FIGURE 4 illustrates the field pattern chart for the MINI treatment unit;

FIGURE 5 illustrates the field pattern chart for the MOYI treatment unit;

FIGURE 6 illustrated the field pattern chart 0048451 for the MAXI treatment unit;

FIGURE 7 is a plan view of the control unit or generator showing the various controls thereon;

FIGURE 8A is a schematic illustration of the basic pulse shaping circuit;

FIGURES 8B and 8C show the current and its derivative with respect to time of the pulse wave form associated with the pulsating system.

FIGURE 9 is a block diagram of the control unit or generator including both sinusoidal and pulsating versions of the generator;

FIGURE 10 is a sequential time diagram showing different control signals generated in both the sinusoidal and the pulsating version of the control unit (generator);

FIGURE 11 is a detailed circuit diagram of the high voltage circuit of the sinusoidal version of the system;

FIGURE 12 is a further block diagram of the control unit (or generator) using a pulse treatment field only;

FIGURE 13 is a detailed circuit diagram of the high voltage circuit of the pulsating version of the system;

FIGURE 14 is a diagram illustrating the electrical current wave forms flowing through the treatment applicators in the sinusoidal field treatment;

FIGURE 15 is a characteristic illustration of the demagnetization curves; and

FIGURE 16 is a diagram illustrating the electrical current wave forms flowing through the treatment applicators in the pulsating field treatment system; the shape of single pulses shown being that illustrated in Figure 8.

DESCRIPTION OF PREFERRED EMBODIMENTS

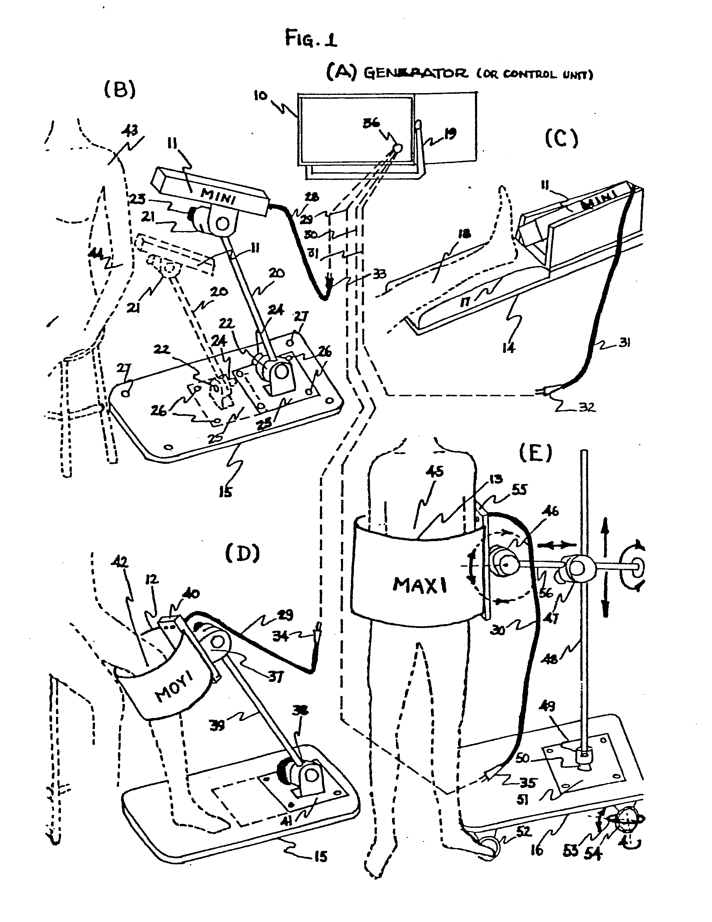

Referring now to the drawings, and more particularly to Figures 1 to 8, there is shown the electro-magnetic therapeutic system of the present invention and comprising different magnetization coils 11 (MINI), 12 (MOYI) and 13 (MAXI) and a control unit or generator 10 of two possible versions, one pulsating 10(a) and one sinusoidal version 10(b), for establishing a predetermined electromagnetic field characteristic in the magnetization coil 12 or 13 or in the surrounding of the magnetization coil 11.

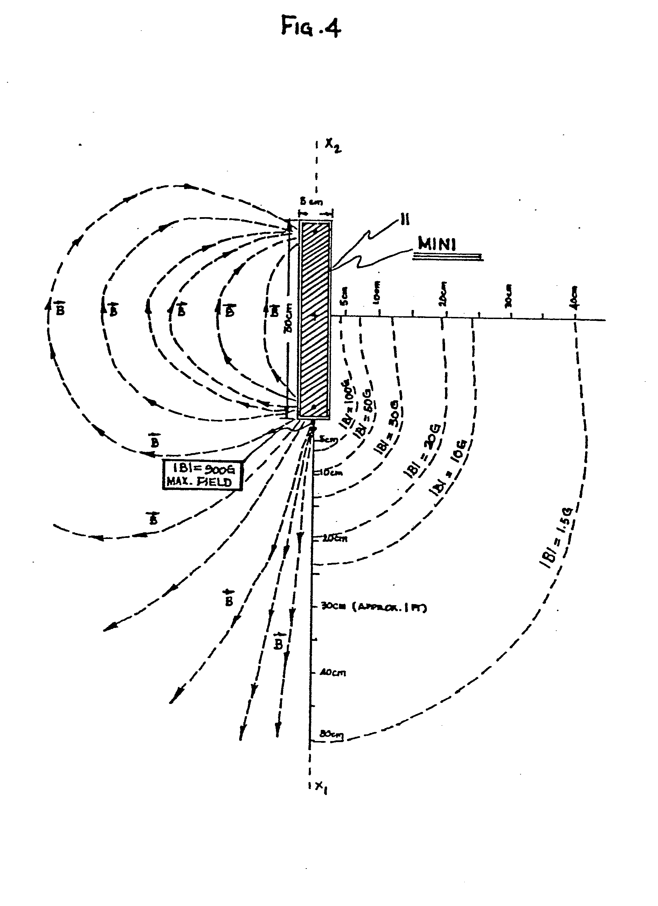

The structural and electrical characteristics of three embodiments of the magnetization coil (MINI Fig. 1B, 1C, MOYI Fig. 1D and MAXI Fig. 1E) are summarized in Table 1 and Table 2 herein, and the field patterns generated in space by these field applicators are well illustrated in Figures 4, 5 and 6 where isomagnetic-field lines are shown (/B/ = 100G, /B/ = 20G, /B/ = 42G, /B/ = 9G, /B/ = 14G, /B/ = 3G, etc.... where /B/ is the maximum intensity of the magnetic field and G means Gauss, the unit of magnetic field, $1G = 10^{-4}$ Wb/m$^2$) and the direction of the field is indicated by arrows in Figures 4, 5 and 6. These "Field Pattern Charts" were built from our experimental measurements using a small (1/4 inch x 1/2 inch) 1200 turn air coil and a known oscilloscope as measuring equipment.

Referring to Figure 4, on the right hand side, the iso-magnetic-field lines are shown for the maximum amplitude setting (A = 10) of the control unit or generator. On the left hand side, the direction of the magnetic field is indicated. For any given point in space, the direction of the field is independent of the amplitude setting of the control unit. Said field lines indicating intensity and direction of field are symmetrical in space with respect to the longitudinal axis $x_1 - x_2$. At one meter away from the MINI treatment unit, the intensity of the magnetic field is of the order of magnitude of the terrestrial magnetic field (below 1G).

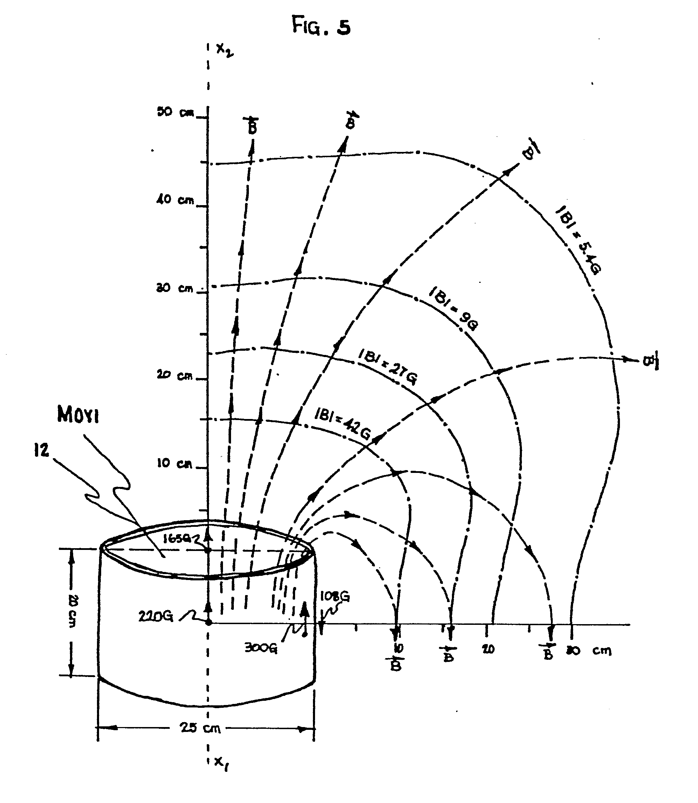

As shown in Figure 5, the isomagnetic-field lines are shown in full lines ($/B/$ = 42 G, $/G/$ = 27 G, and $/B/$ = 9 G) for the maximum amplitude setting (A = 10) of the control unit or generator. The direction of the field is indicated by arrows. For any point in space, the direction of the field is independent of the amplitude setting of the control unit. Said field lines indicating intensity and direction of field are symmetrical in space with respect to the longitudinal axis $x_1 - x_2$. At one meter away from the MOYI treatment unit, the intensity of the magnetic field is of the order of magnitude of the terrestrial magnetic field (below 1 Gauss).

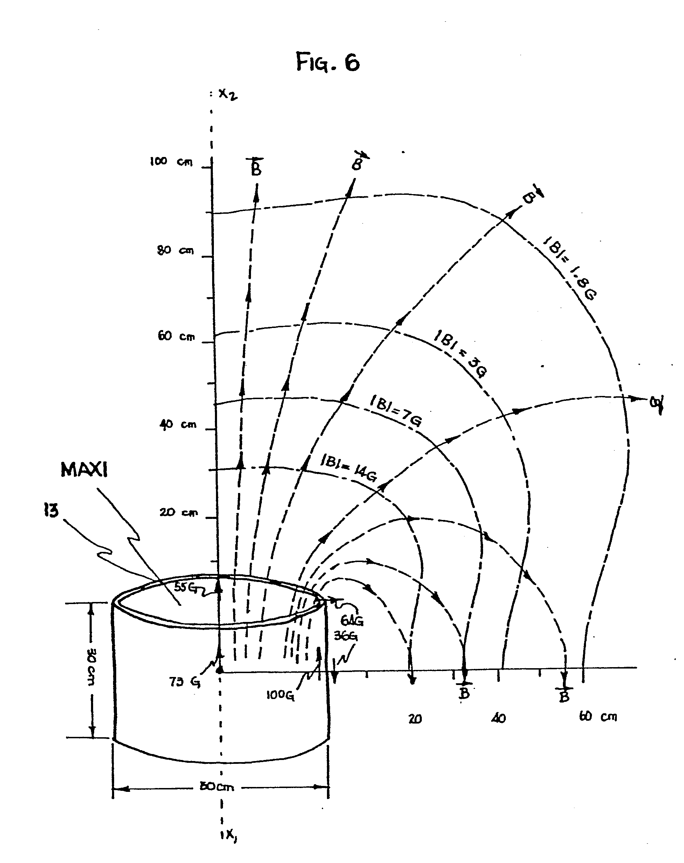

In Figure 6, the isomagnetic-field lines are shown in full lines (/B/ = 14 G, /B/ = 7 G, and /B/ = 3 G), for the maximum amplitude setting (A = 10) of the control unit or generator. The direction of the magnetic field is indicated by arrows. For any point in space, the direction of the field is independent of the amplitude setting of the control unit. Said field lines indicating intensity and direction of field are symmetrical in space with respect to the longitudinal axis $x_1 - x_2$. At one meter away from the MAXI treatment unit, the intensity of the magnetic field is of the order of magnitude of the terrestrial magnetic field (below 1 Gauss).

TABLE 1

STRUCTURAL CHARACTERISTICS OF THE TREATMENT
UNITS:  MINI, MOYI AND MAXI

| Name of treatment unit | Shape of treatment unit | Outside diameter of treatment unit (inches) | Length of treatment unit (inches) | Winding (gauge $N_0$. 12 copper wire or equivalent) of treatment unit |
|---|---|---|---|---|
| MAXI, 13 | Hollow cylinder (1/4" thickness approx.) | 20" | 12" | One 60 turn row spread on 10 5/8" long |
| MOYI, 12 | Hollow cylinder (3/8" thickness approx.) | 10" | 8" | One 73 turn row spread on 6" long, and a second row:  12 turns spread on 6" long |
| MINI, 11 | Long rectangular shape | N.A. | 13 3/4" x 2 1/4" x 2 1/4" (external dimensions) | One 134 turn row<br><br>Core:  high efficiency laminated steel or equivalent: 1" x 1" x 12" core.  Thickness of steel laminates:  1/32" |

TABLE 2

ELECTRICAL CHARACTERISTICS OF TREATMENT APPLICATORS
PREFERRED "OPTIMAL" VOLTAGES AND CURRENTS AND MAXIMUM
FIELD GENERATED BY MINI, MOYI AND MAXI

| Electrical parameters of treatment units | | | Ri-2000 S (Sinus option) | | Ri-2000 P (Pulsatile option) | | Ri-2000 S Ri-2000 P |
|---|---|---|---|---|---|---|---|
| Treatment unit | $r_L$* $(\Omega)$ | Inductance L (mH) | Voltage across coil (Volts) peak-peak | Current through coil (Amp) peak-peak | Voltage across coil (Volts) peak | Current through coil (Amp) peak | Maximum field (Gauss) |
| MINI | 0.06 | 2.32 | 35 | 40 | 150 | 20 | 900 |
| MOYI | 0.38 | 2.06 | 35 | 45 | 150 | 21 | 300 |
| MAXI | 0.55 | 2.21 | 35 | 42 | 150 | 21 | 100 |

* $r_L$ = The D.C. electrical resistance of the winding.

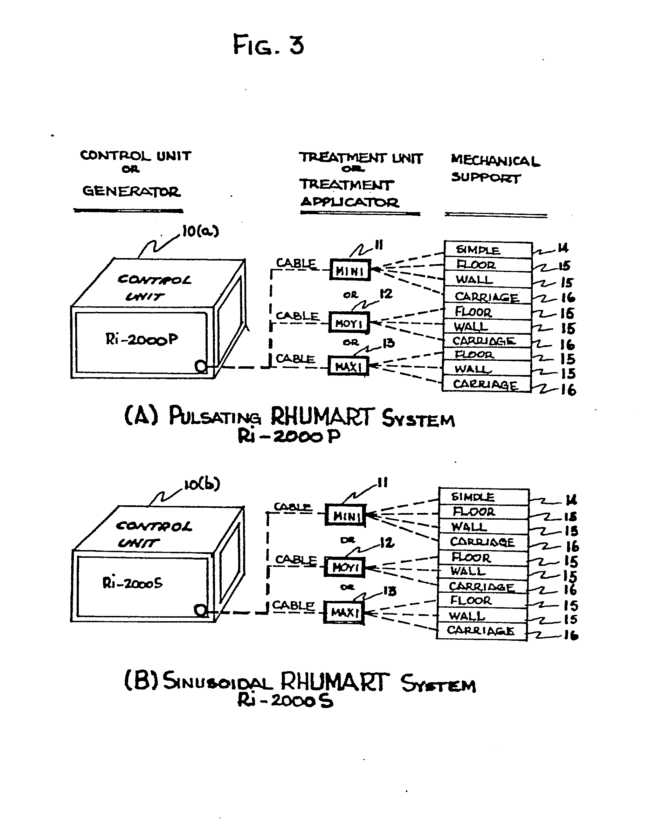

The field applicators, 11, 12 and 13 are used for treatments such as shown in Figures 1A, B, C, D and E illustrations, and such as further illustrated by the different combinations of generators, treatment applicators and mechanical supports shown in Figures 3A and B illustrations. The MOYI and MAXI treatment applicators are air coils and the MINI treatment applicator is a core coil as further described in Tables 1 and 2 hereinbefore.

The best characteristics of the three treatment applicators resulted from a systematic optimization study where the purpose was to achieve the highest field possible with the smallest and lightest generator possible while allowing a great variety of sinusoidal and pulsating field patterns and with a further object of creating electrically and mechanically compatible and/or interchangeable treatment applicators. All this was achieved with the characteristics given in Table 1 and Table 2, and more particularly, interchangeability was achieved by designing treatment coils having approximately the same inductance L (approx. 2 millihenry for MINI, MOYI and MAXI). The voltage and current levels were also optimized in order to reduce the number of turns in the windings (reducing cost and weight) while preventing excessive currents which would cause unacceptable temperature rise of treatment coils when used with maximum power.

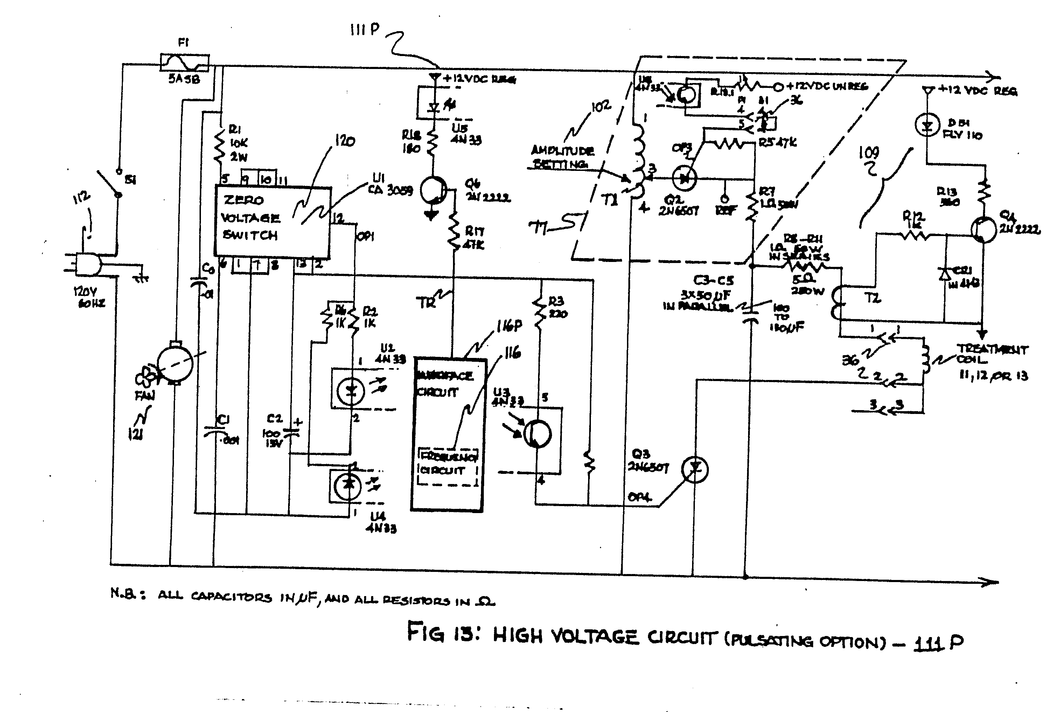

In a preferred embodiment of the pulse shaping circuit, Figure 8A, well defined relationships exist between the different components of this circuit and the desired current pulse shape: e.g., $t_m = 2L/R_L$; $C = 4L/R_L^2$; $i_m = -(R_L/2L) C V_o/e$; $R_L = r_L + R_{Damping}$; and $t_i = 2t_m$; where $i_m$ = maximum current flowing through treatment applicator; $t_m$ = period of time between beginning of pulse and time of maximum current $i_m$; L is the inductance of the treatment applicator; $R_L$ = total series resistance in the capacitor discharging circuit, including the D.C. resistance ($r_L$) of the treatment applicator in series with all damping resistances ($R_{Damping}$) of the discharging circuit; C = the capacitance of the capacitor(s) which is repeatedly charged and discharged by closing and opening $K_1$ and $K_2$ respectively so that $K_1$ is closed when $K_2$ is open and conversely. $K_1$ and $K_2$ are Silicon Controlled Rectifiers. The charging circuit 77 is fed by an alternating current supply, 50 or 60 Hz 115 or 230 Volts as further illustrated in Figure 13. $V_o$ is the maximum voltage to which the capacitor C is charged; "e" is the base of the neperian logarithm (e = 2.71828), $t_i$ is the period of time from the beginning of the current pulse to the time where the second derivative of the current $i_c$ is equal to zero; $\dfrac{d^2 i_c}{dt^2} = 0$, then $t = t_i = 2t_m$ as illustrated in Figure 8C of the present Figure. It can be shown that: $i = -(R_L/2L)^2 CV_o t e^{-(R_L/2L)t}$ where t is the time and i is the current flowing through the treatment coil, for the critical damping design.

All three treatment applicators 11, 12 and 13 can be connected to any of the two versions of the generators 10(a) and 10(b) using a jack connector 32, 33, or 35 which fits its female jack connector 36. Mechanical versatility is still increased by the use of four different types of modular supports which are constructed with a minimum number of standardized parts such as an articulated joint 21, 22, 37, 38 and 46; non-magnetic stainless steel 1 1/4 inch rods 20, 39, 56 and 48 (bigger rod, 1 1/2 inch), galvanized 8 x 8 inch aluminum plates 25, 41, and 51 are fixed with non-magnetic stainless steel screws 26, base plates 15, 16 which can be made of wood covered with suitable material. Casters 52, 54 can be fixed to the base of different mechanical supports 15 and 16, the rear wheels 54 are equipped with a braking mechanism 53 and they are mounted on a rotating base. The base 15 and 16 of mechanical supports can also be fixed to a wall or to the ceiling of a treatment room. The base plate 25, 41 or 51 can be fixed in the middle of the support base 15 or 16 when mechanical stability is preserved for a given set-up of mechanical support.

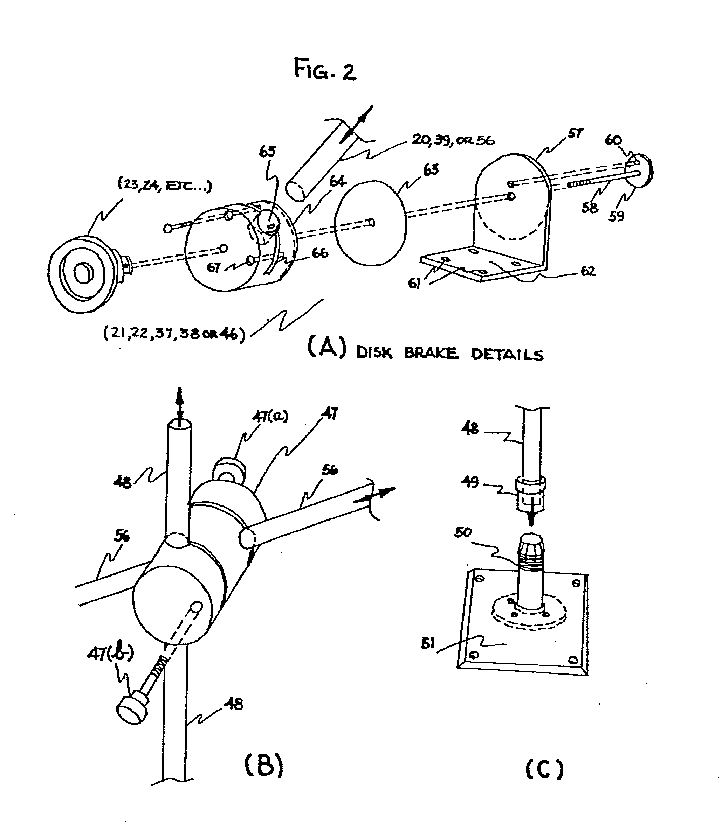

Figure 1C shows a simple mechanical support 14 composed of a comfortable cushion 17 and a simple rect-angular box to keep the treatment applicator 11 in the desired position. Different height adjustment inserts (small wedges or boards) can be placed under MINI, 11, for adjusting its level. Support plates 40 and 55 hold the coil 12 and the coil 13 to the base 62 of the articulated joint 37, 46, illustrated in Figure 2.

This joint 21, 22, 37, 38 or 46 is composed of a break holder 57, a large flat end screw 58, 59 with a lock pin 60, a thin frictional disc 63, a cylindrical part 64 with a hole 65 to receive the rods 20, 39 or 56. These rods can be rigidly fixed in the hole 65 by tightening two allen screws 67 which cause the slot 66 to narrow. A handle 23, 24, is used to adjust the brake pressure by pressing all parts of the disc brake together when the handle 23, 24 is screwed on the flat end screw 58, 59.

Figure 2B shows the details of a connector 47 which serves to hold the rods 48 and 56 at perpendicular angles between each other, while allowing the rods to be partially moved and locked in desired positions. The adjustment is provided by flexible slots adjusted with pressure screws 47(a) and 47(b); a mechanical coupling 49-50 holds the rod 48 to the base plate 51 (see Figure 2C). The screw nut 49 is used to tighten the threaded slotted rod end 50 onto the vertical rod 48.

Figure 3 is a block diagram showing different combinations of generators 10(a) or 10(b), treatment applicators 11, 12 or 13 and mechanical supports 14, 15, 16 of the Electromagnetic Therapy System.

Figure 4, Figure 5 and Figure 6 show the three field applicators 11, 12, and 13 with their respective field pattern chart as described hereinbefore.

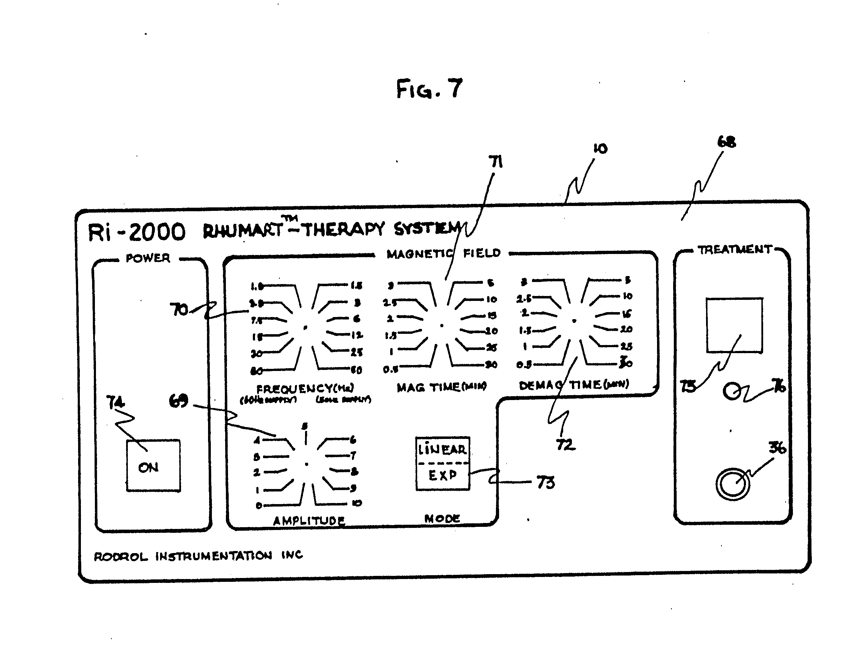

Referring now to Figures 7 to 16, there will be described the field generator 10, 10(a) and 10(b). The system is intended for medical use and for multiple treatments. The treatment is generated by a magnetization coil 11, 12, 13 properly coupled and fed by a sinusoidal or a pulsating generator 10(a) and 10(b). Most of the characteristics of the magnetic field created by the system are selected by the controls on the front panel of the generator 10 as illustrated in Figure 7.

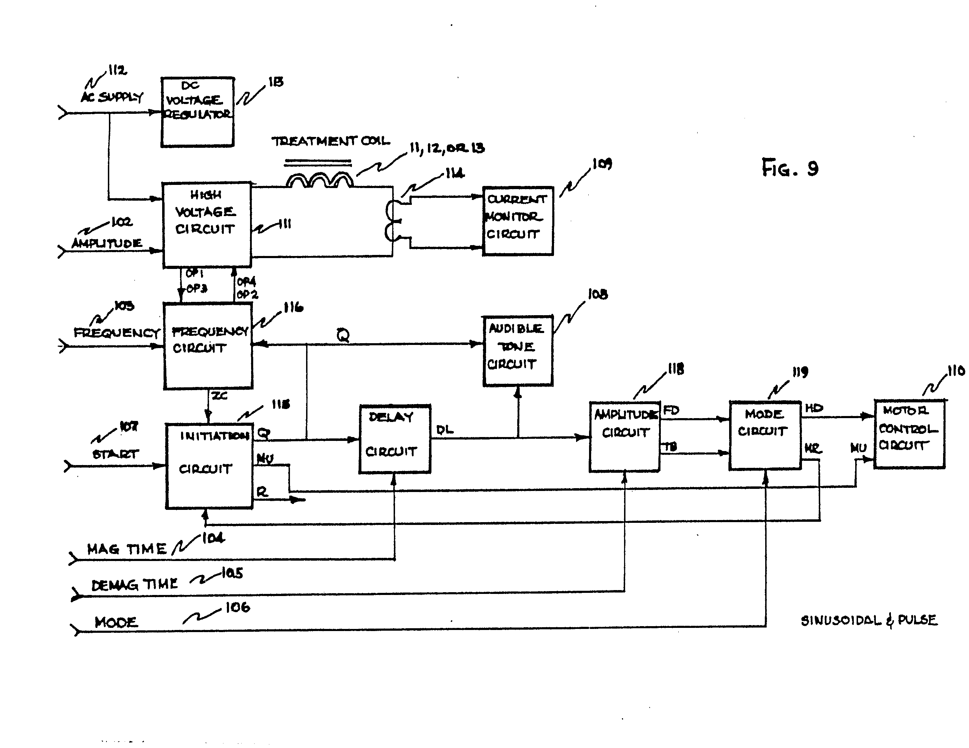

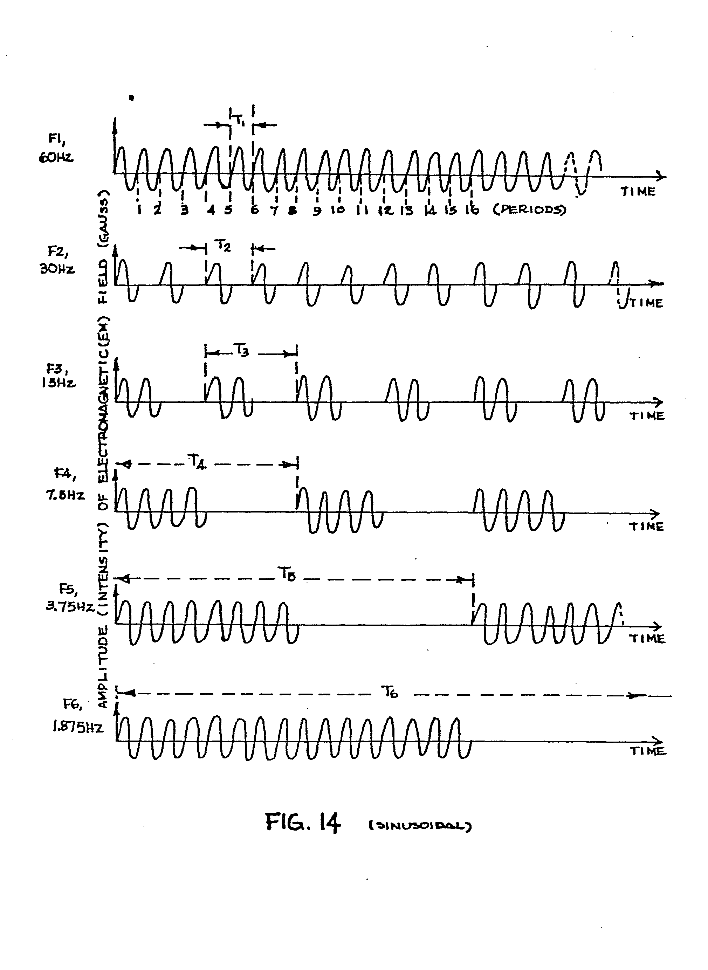

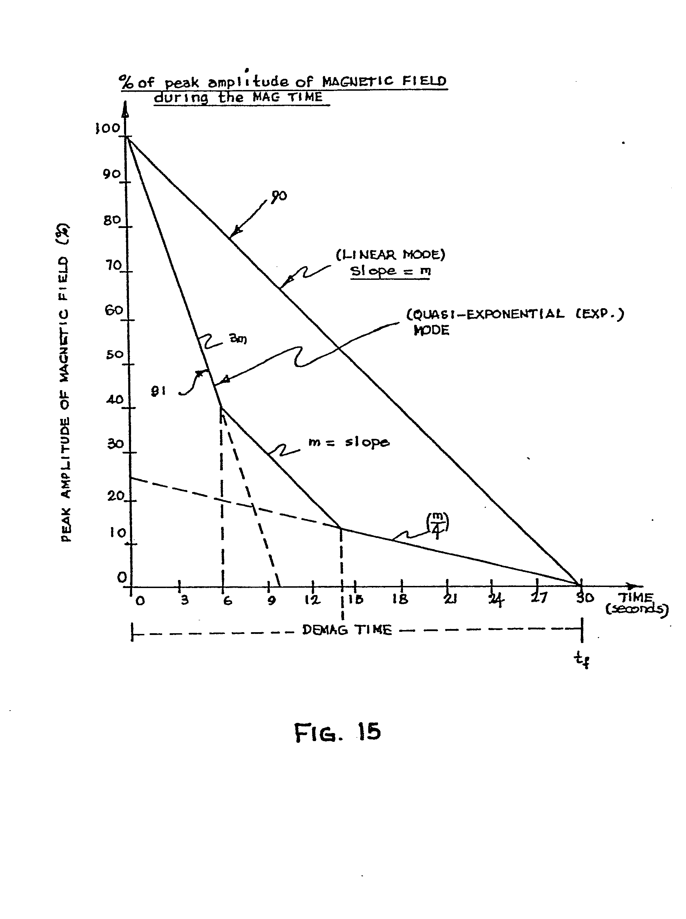

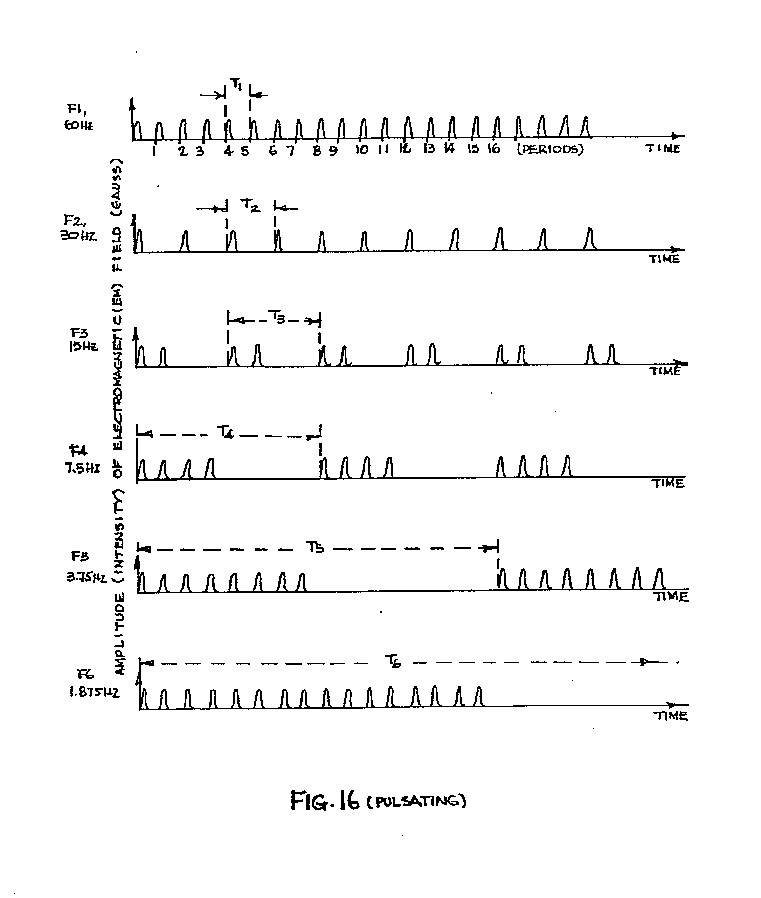

In a first option of the system, the electromagnetic field is pulsed (Figures 8, 12 and 13) and in a second option, the electro-magnetic field is sinusoidal (Figures 9, 11 and 15). As shown in Figure 7, the magnetic field has five characteristics which are adjustable by the controls on the panel 68 of the control unit 10. The first control 69 controls the amplitude or intensity of the electro-magnetic field from 0 to 100% as herein shown in increments of 10%. The control 70 controls the frequency of interruption of the fundamental signal of the electro-magnetic field in the order of 1, 2, 4, 8, and 16 cycles of the periodic field, see Figures 14 and 16 for detailed illustration, with an interruption time respectively equal to 1, 2, 4, 8 and 16 cycles of the periodic field, see also Figures 14 and 16. The magnetization time is set by the control 71 and at the termination of the magnetization time, the second phase or the demagnetization phase of the treatment takes place. This demagnetization phase has a time period set by the control 72. The demagnetization can be made in accordance with a linear or quasi-exponential characteristic as will later be described with reference to Figure 15.

The switch 73 sets the desired characteristic of the demagnetization phase either for linear or exponential demagnetization. Switch 74 is an "ON" switch for the system while switch 75 is the switch which starts the treatment and the various timers are synchronized with the switch 75. Indicator light 76 indicates the start and termination of the treatment and that current effectively flows through the treatment applicator 11, 12 or 13. A jack 36 is provided to connect the magnetization coil 11, 12 or 13 to the generator 10.

Referring again to Figure 8 which is an illustration of the pulse shaping curcuit and pulse wave forms 78 and 79 of the pulsed magnetic field as discussed above, a specific embodiment of the present invention is represented by the following set of values for $t_m$, L, $R_L$, C and $i_m$:

choosing $t_m$ = 0.5 msec (for physiological reasons); and

L = 2 m Henry (found to be appropriate for the present invention); then

we can calculate $R_L$ = $2L/t_m$ = 8$\Omega$; and

$C = 4L/R_L^2 = 125 \,\mu F$; and

$i_m = -(R_L/2L)\ C\ V_o/e = 18.45$ Amperes.

This set of values represent what is named the critical damping design for the given values of $t_m$ and L. Other embodiments of the present invention can easily be reached by using the same set of simultaneous equations for desired values of $t_m$ and L. It is intended that the numerical value of the components $R_L$, L and C of the pulse shaping circuit can be changed so as to cause the peak amplitude of the said current pulse 78 to decrease by not more than 50% of that resulting from the critical damping design defined above and the resulting design is included in the present invention and is an embodiment of it.

Referring now to Figure 9, the connection 107 connects to the switch 75 to start the treatment by operating an initiation circuit 115. The treatment will be effected as set by the controls on the generator 10. An audible circuit 108 is actuated during the period of treatment. The current monitor circuit 109 operates the indicator lamp 76 on the console 10. In the second phase of treatment during the demagnetization time, the amplitude is automatically varied by means of an amplitude circuit 118, a mode circuit 119, and an electromagnetic system 110 including a motor (not shown) coupled to a variable transformer incorporated in the "High Voltage Circuit". The interruption of the electromagnetic field to produce pulse signals is effected by means of a power semi-conductor (Silicon Controlled Rectifier, SCR) in the high voltage circuit 111. The entire control unit is fed by a 60 cycle 115 volt supply or 50 cycle 230 volt supply at 112. It is the frequency of the A.C. voltage 112 that determines the fundamental frequency of the magnetic field treatment. A 12 volt D.C. voltage regulated and unregulated is also provided to feed the various electronic and electro-magnetic circuits in the apparatus. The various controls 69, 70, 71 and 72 are represented in Figure 9 at inputs 102, 103, 104 and 105. The mode switch 73 is represented at 106.

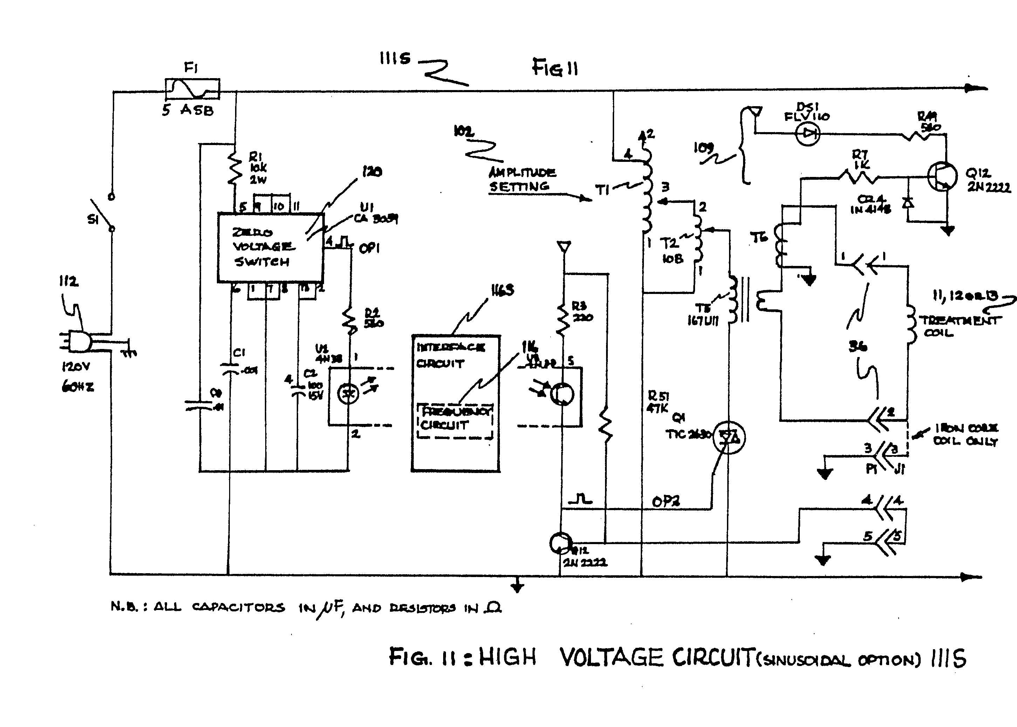

Referring to Figures 9 and 11, the high voltage circuit 111 (or 111S) feeds the current to the magnetization coil 11, 12 or 13 and includes a variable auto-transformer T1 which permits to adjust the maximum current for a given treatment to the desired value. A voltage transformer T5 lowers the voltage in a manner whereby to obtain a current of high intensity in the coil 11, 12 or 13. A semi-conductor of the TRIAC type Q1 permits the interruption of the current in accordance to the desired magnetic field pattern. This TRIAC Q1 is controlled by a signal OP2.

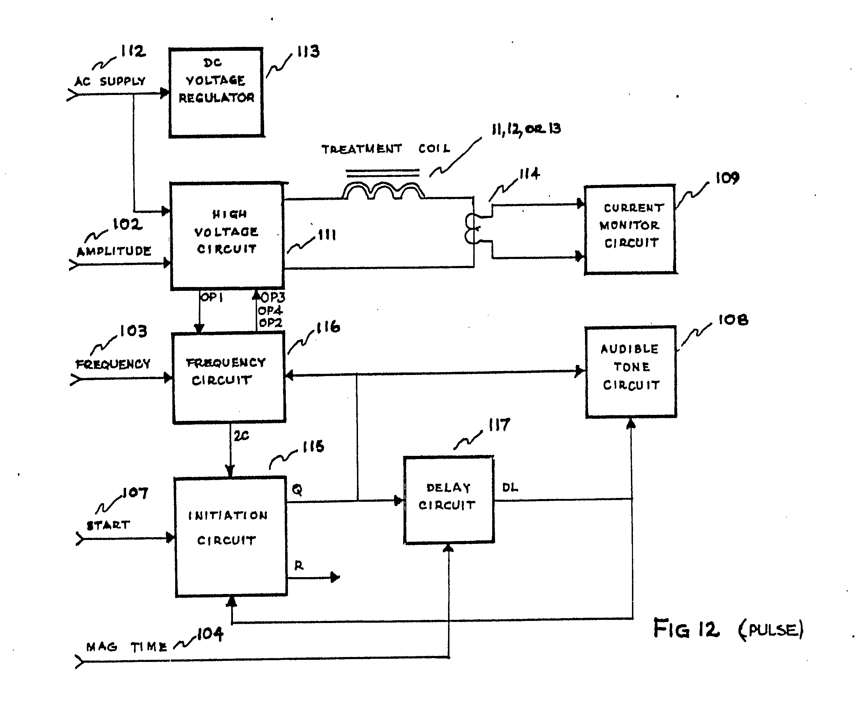

Referring to Figures 12, 13 and 8, in the application of a pulse magnetic field, a capacitor C3-C5 is charged with the current in the secondary of the auto-transformer T1. The capacitor C3-C5 is then discharged in the treatment coil 11, 12 or 13 and produces a pulse current of high intensity. This is achieved by means of an SCR semi-conductor Q3 controlled by a signal OP4. This signal corresponds to the signal OP3 of the capacitor charging circuit 77 except that it is delayed and that the current pulses 78 correspond to the negative cycle of the sinusoidal signal. A detector circuit named zero voltage switch 120 is used to detect the near-zero tension of the A.C. supply 112 and to generate the synchronization signal OP1 as shown in Figures 11 and 13. A small cooling fan 121 is used to cool down the damping resistors R8-R11 ($5\Omega$, 250W). When the system is used with a voltage of 230 volts at 50 Hz, an extra input-auto-transformer is used to reduce this voltage to 115 volts 50 Hz.

The D.C. voltage regulator circuit 113 utilizes a transformer which reduces the voltage as is necessary to supply the circuits. A regulator is also used to obtain a continuous voltage of 12 volts and an unregulated 12 volts D.C.

The current monitor circuit 109 includes a current transformer 114 (T6 and T2 on Figures 11 and 13) which feeds the indicator lamp 76 which permits visual indication that current flows through the magnetization coil 11, 12, 13.

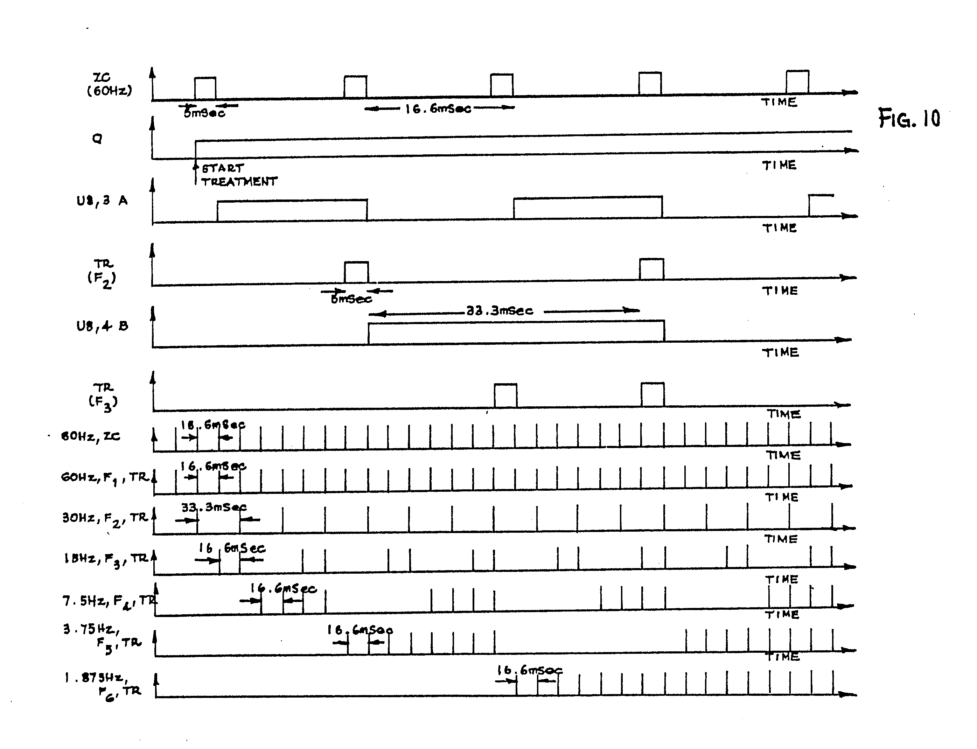

Referring now to Figures 9 and 10, the initiation circuit 115 is operated by the start switch 75 and is placed in operation by the first pulse of the signal ZC which is similar to the signal OP1. The signal MU at one of the outputs of the circuit 115 determines the speed of the motor acting to position T2 (Figure 11). The signal Q commands the first phase of the treatment (Figures 9 and 10), that is, the MAG TIME phase. The signal MR appears at the end of the DEMAG TIME and resets all the circuits and the position of the auto-transformer T2 (Figures 9, 10 and 11).

Referring to Figures 9 to 13, the frequency circuit 116 (116S and 116P on Figures 11 and 13) produces an interruption of the magnetization current at a predetermined frequency 103. It generates a series of pulses TR (Figure 10). The signal OP2 (or OP4) is retarded relative to the signal OP1 (and OP1 and OP3 in the pulsating case) in such a manner to interrupt the passage of the current in the treatment coil as illustrated in Figures 10, see ZC, F1, F2...F6 and in Figures 14 and 16, see F1, F2,...F6.

The delay circuit 117, Figure 9, generates the time period of the MAG TIME of the treatment as predetermined by the setting on the control 71. At the end of MAG TIME, the signal DL commands the other circuits.

The audible tone circuit 108 produces two different frequencies in accordance with the various sections of the treatment. It is operated by the signal Q. The change of the tone of the signal is controlled by the signal DL.

The amplitude circuit 118 generates a signal FD of a predetermined frequency in accordance with the function of the DEMAG TIME 105 and set by the control 72. This circuit is controlled by the signal DL during the second phase or the DEMAG TIME of the treament. This

- 33 -

circuit also generates a time base TB. The time unit of this time base is variable in accordance with the setting of the DEMAG switch 72, and allows for generating different demagnetization curves as illustrated in Figure 15, where DEMAG TIME is equal to $t_f$, 30 seconds for the case illustrated.

The mode circuit 119 generates a linear curve or a quasi-exponential curve of initial amplitude equal to the peak amplitude of the magnetic field generated during the MAG TIME. This choice is established by the mode switch 73. The time base signal will modify the frequency signal FD in such a way as to produce a second signal MD of which the frequency will control the speed of the motor in circuit 110. This motor is a step-by-step motor and the direction of rotation of this motor is determined by the signal MU or MD. This motor is fed by a 12 volt supply and controlled by means of transistors. This motor operates a variable auto-transformer T2 which in turn modifies the current in the magnetization coil 11, 12 or 13 whereby to demagnetize the coil in accordance with the preset mode characteristic 73, 90, 91 (Figs. 7 & 15).

Figure 12 includes the same component parts of the circuit described in Figure 9 with the exception that it is restricted to the pulse mode, whereas in Figure 9, there is shown both the pulse and the sinus modes.

Referring now to Figure 14, there is shown an illustration of wave forms F1, F2...F6 for the sinus electro-magnetic field treatment. As shown, the basic wave form is a sinusoidal wave form which is either a 60 or 50 HZ signal depending on the frequency supply of the input voltage 112. When a 50 Hz supply is fed into the system, then the resulting treatment frequencies F1, F2, ...F6 will be equal to 50 Hz, 25 Hz 12.5 Hz, 6.25 Hz, 3.125 Hz and 1.56 Hz, for both sinus and pulse options.

Figure 15 is an illustration of the characteristic of the demagnetization curves when the system is utilized in the linear or the quasi-exponential mode. The characteristic of the linear mode is shown at 90 and the quasi-exponential mode is shown at 91. The DEMAG TIME $t_f$ = .5, 1, 1.5 min., etc. as shown on the control 72 of Figure 7.

Figure 16 shows the electromagnetic field patterns for the pulse option. The shape of individual pulses is as described above, see Figure 8 and its description hereinbefore.

The following is an actual specification of the system realized in accordance with the present invention.

## Ri-2000 RHUMART-THERAPY SYSTEM

### S P E C I F I C A T I O N S

#### SUMMARY

Voltage supply:   110 or 220   VAC, 60/50 Hz Max. Power requirement:
- Ri-2000S:   approx. 330 W
- Ri-2000P:   approx. 550 W Fuse:
- Ri-2000S:   approx. 5 Amp.
- Ri-2000P:   approx. 5 Amp.

Field intensity*:   adjustable 0-100 gauss (peak), with MAXI.

Basic Sinusoidal or Pulse Frequency:   60/50 Hz Treatment-modulation frequencies:
- 60 Hz, 30 Hz, 15 Hz, 7.5 Hz, 3.75 Hz and 1.875 Hz;
- or: 50 Hz, 25 Hz, 12.5 Hz, 6.25 Hz, 3.125 Hz, 1.56 Hz.

(depending on the basic frequency:   60 or 50 Hz) Duration of treatment:
- MAG. TIME: 0.5, 1.0, 1.5, 2.0, 2.5, 3.0, 5.0, 10, 15, 20, 25, and 30 min.
- DEMAG TIME: 0.5, 1.0, 1.5, 2.0, 2.5, 3.0, 5.0, 10, 15, 20, 25, and 30 min.

(Duration of treatment = MAG. TIME + DEMAG. TIME)

Treatment units:
- MINI   :   6 x 6 x 35 cm
- MOYI   :   25 cm I.D., 20 cm long
- MAXI   :   50 cm I.D., 30 cm long

Size: Control Unit: 16 x 29 x 33 cm.

* Field intensity:
- MINI : 0-900 gauss (peak)
- MOYI : 0-300 gauss (peak)
- MAXI : 0-100 gauss (peak)

See Figures 4, 5 and 6 for details on Field Patterns.

The operation of the   system   and the general working instructions are as follows:

a)   Sequence of operating instructions:

- Select the proper treatment unit (MINI, MOYI or MAXI) and plug it into the control unit (bottom right of front panel). (See the note below Table 3);

- Plug the control unit (Ri-2000S or Ri-2000P) to the power outlet and press the POWER switch to "ON" position (switch lights up);

- Place the patient within the range of the chosen treatment unit (MINI, MOYI or MAXI). When MOYI or MAXI are used, the head or a single limb can be placed into these cylincrical treatment units;

- Select the proper treatment parameters using the TABLE OF INDICATIONS (Table 3) all information contained in the present document, and seek professional advice when in doubt. The range of treatment parameters is as follows:

    Amplitude:    from 1 to 10 (see Figures 4, 5 and 6 for details)

    Frequency:  from 1.85 Hz to 60 Hz or 1.56 Hz to 50 Hz Time (Ri-2000P):   from 0.5 to 30 min.

    MAG TIME (Ri-2000S): from 0.5 to 30 min.

    DEMAG TIME (Ri-2000S): from 0.5 to 30 min.

    MODE (Ri-2000S):  LINEAR or EXPONENTIAL

- Press the START-TREATMENT push button (which lights up as treatment starts). A small red light (below the START switch) indicates that current is effectively flowing through the treatment unit, when the amplitude, A, is above 10% of maxiumum. When the frequency is below 20 Hz, this red light flickers;

- During the entire time of treatment, an audible tone is generated; at the end of the treatment, the amplitude of the magnetic field is automatically

- 27 -

decreased to zero, and the audible tone is auto-matically turned off.

- Remove the patient from the treatment unit (MOYI or MAXI) or away from the MINI treatment unit.

- Turn the amplitude control knob to zero, and turn the POWER switch OFF.

B) <u>General working instructions</u>:

- Patients should not be treated late in the evening because, occasionally, disturbed sleep may occur.

- During the treatment, the patient should be placed in a comfortable position.

- The patient is usually sitting during treatment.

- The patient does not need to undress for treatment with the RHUMART-therapy system.

- Watches and other ferromagnetic materials worn by the patient should be removed, as they disturb the magnetic field.

- Other metallic objects do not need to be removed by the patient.

- The treatment units MINI, MOYI or MAXI should not be lined with blankets or other isolating material, as the waste heat could not be carried away, and overheating might result. Moderate heating of the treatment unit is normal after a long period of treatment with high dosage. The system can be operated without interruption over a long period of time. It may be left in operation continuously although it is not recommended.

- The first 5 to 10 treatments should be carried out on consecutive days if possible, or at least every second day. The subsequent treatments may be carried out every third day or less often.

- Not all patients respond in an equally positive way

0048451

to the sinusoidal (Ri-2000S) or pulsating (Ri-2000P) magnetic field.

- If the patient does not respond to the magnetic field after the 4th or 5th treatment, and if he/she does not feel any effect, you should always consider to to use the Ri-2000S control unit if the Ri-2000P was used for this patient and reversely. After 3 or 4 treatments, you should decide whether there is any reason for continuing the therapy. However, there have been cases where the patients felt a positive effect only after the 15th or even the 20th treatment. This is sometimes the case with chronic degenerative diseases.

- A slight increase in the complaint after the first two or three treatments is not unusual, since this is the initial adaptive phase of the treatment course, and it is not a reason to interrupt the therapy, because improvement will probably follow in most cases. Such an adaptive phase is known to occur in other forms of therapy.

- Make sure that the activation of a latent focus does not lead to complications. If, after the 1st to 3rd treatment, continuous pain is felt in the region of the body where the focus happens to be, then stop the treatment course.

## Table 3

### TABLE OF INDICATIONS: (parameters suggested with the Ri-2000P system)**

| ORTHOPEDICS – SPORT MEDICINE – SURGERY – GERIATRICS–DENTISTRY–RHEUMATOLOGY | | |
|---|---|---|
| 1.*distorsions contusions 100*;50 to 60;20 | 5. *osteoporosis pseudoarthrosis 80*;50 to 60;20 | 9. rheumatic diseases (chronic) 60*;25 to 30;15 |
| 2.*fractures dystrophy 100*;50 to 60;20 | 6. rheumatoid spondylitis 80*;50 to 60;20* | 10. Cervical, lumbar, thoracic spinal syndrome – acute rheumatic diseases. 40*;3 to 5;15 |
| 3.*traumatic injuries dislocations 100*;50 to 60;20 | 7. periarthritis epicondylitis 60;3 to 4;15 | 11. ulcus cruris post radiation therapy 60*;12 to 15;20 |
| 4. musculoskeletal degeneration 80*;50 to 60;20 | 8. treatment after tooth extraction 80*;25 to 30;15 | |

| NEUROLOGY | | |
|---|---|---|
| 1. brachial neuralgia intercostal neuralgia 80*;50* to 60*;20* | 3. head neuralgia disturbed sleep 60*;3 to 5;10 | 5. migraine 30*;1.5 to 2;10 |
| 2. lumbalgia 80*;12* to 15*;15 | 4. depressions 60*;6 to 8;15 | |

| INTERNAL MEDICINE – GYNECOLOGY – REHABILITATION – OTHER INDICATIONS | | |
|---|---|---|
| 1. nephritis 100*;50* to 60*;15* | 5. cardiac insufficiency nervous stomach 50*;12* to 15*;15 | 9. vegetative dysfunction 40;12 to 15;20 |
| 2. metabolic insuff. old-age diabetes 80*;12 to 15;20 | 6. chronic sinusitis cerebral circulatory disorders 40*;12* to 15*;10 | 10. chronic gastric ulcers 60*;3 to 5;15 |
| 3. hypotension various origin 80*;50* to 60*;15 | 7. bronchitis 60*;3 to 5;15 | 11. acute sinusitis 30;3 to 5;10 |
| 4. insufficient liver function 40;3 to 5;15 | 8. bronchial asthma ulcerative colitis 40;3 to 5;15 | |

NOTE:

This indication chart should only be used for the RHUMART-THERAPY SYSTEM (Ri-2000P). The figures are only suggestions. * The asterisk (*) means gradual increase i.e., the first treatment should be carried out at 30% of the indicated value, the second at 70% and the third treatment at 100%. In case of several indications, one should always start with the indication that requires the lowest intensity and frequency. The first figure indicates the intensity in Gauss, the second figure indicates the frequency in Hertz, and the third indicates the period of treatment, in minutes.

(The 3 treatment units have been named MAXI, MOYI and MINI)

## Which treatment unit should be used:

MAXI: can be used for all recommended indications: The conversion of amplitude (A) to Gauss is indicated on MAXI itself. (see Figure 6 for the FIELD PATTERN CHART).

MOYI: can be used for all recommended indications where the treatment unit need not be placed around the torso or around the waist of adults: The con-

version of amplitude (A) to Gauss is indicated on MOYI itself. (see Figure 5 for details).

MINI: can be used in all localized disorders (focus) and in systemic disorders by treating via the hands and feet: The conversion of amplitude (A) to Gauss is indicated on MINI itself for the maximum field near the treating end of MINI. See the "FIELD PATTERN CHART" (Figure 4) for the amplitude (intensity) and direction of the field around the MINI Treatment Unit.

** For the Ri-2000S system, it is suggested to use the same parameters as those used with the Ri-2000P system, except for the period (or time) of treatment. With the Ri-2000S system, the total time of a treatment is divided in two parts: the MAG TIME and the DEMAG TIME; and it is suggested to use 25% of the time indicated in the above Table for the MAG TIME and the same value for the DEMAG TIME. Therefore, the total time of treatment suggested with the Ri-2000S is 50% of the treatment time indicated in the above Table.

Table 4

Results obtained by different therapeutists using low
frequency electromagnetic waves (of the type generated
by the Ri-2000P)*

| INDICATIONS For each indication, $30 \leqslant \binom{\text{number of}}{\text{patients}} \leqslant 60$. | | Results (%) * | | | | |
|---|---|---|---|---|---|---|
| | | Very good | Good | Satisfactory | No result | Negative result |
| | | (%) | (%) | (%) | (%) | (%) |
| RHEUMATHOLOGY | Rheumatic diseases (chronic) | 10 | 70 | 25 | 10 | 0 |
| | Rheumatic diseases (acute) | 10 | 55 | 20 | 5 | 0 |
| | Chronic inflammatory joints | 0 | 55 | 35 | 5 | 0 |
| | Chronic polyarthritis | 5 | 35 | 40 | 10 | 5 |
| | Periarthritis | 10 | 35 | 35 | 20 | 0 |
| | Epicondylitis | 15 | 40 | 35 | 10 | 0 |
| ORTHO-PEDICS | Traumatic injuries | 10 | 60 | 20 | 5 | 0 |
| | Contusions distorsions | 30 | 50 | 15 | 5 | 0 |
| | Fractures | 25 | 50 | 10 | 10 | 0 |
| | Osteoporosis | 15 | 20 | 50 | 20 | 0 |
| NEURO-LOGY | Neuralgia | 25 | 35 | 25 | 15 | 0 |
| | Lumbalgia | 15 | 55 | 30 | 0 | 0 |

* Note:    The figures in this table were rounded to
the nearest 5%. These findings are com-
patible with those of our clinical evaluation
results.

## APPLICATION EXAMPLES

1.    TREATMENT

Important:   In many cases, the biological effects which results from weak electromagnetic fields cannot be generated by strong fields. Therefore, it is recommended to start the therapy with weak fields and low frequencies, and gradually increase them from treatment to treatment. At the end of the therapy, it is recommended to decrease these parameters (amplitude and frequency) gradually in the last few treatments. The Ri-2000S sinusoidal field system is particularly indicated to complete a treatment course, as the DEMAGNETIZATION phase (during DEMAG TIME) of the treatment automatically causes the amplitude of the field to decrease at a desired rate (0.5 min. to 30 min. period) for a choice of two different decay modes (LINEAR or EXPONENTIAL).

i) First phase of treatment:

During this intial phase, the treatment should always be carried out with a 30% initial amplitude, frequency and time when the asterisk (*) is shown in the TABLE OF INDICATIONS (Table 3); the second treatment can reach 70% of the desired amplitude, frequency and time; only the third treatment should be carried out with the desired maximum values given in the TABLE OF INDICATIONS.

The subjective feelings of the patients should be used as an indicator.

It is important to use all information available in the present document in order to determine optimum dosage, as this effort will be rewarded by much faster results, taking advantage of previous clinical experiences.

ii) Second phase of treatment:

This is RHUMART therapy using the maximum recommended dosage for a particular application (see Table 3).

The duration of treatment varies from 0.5 to 30 minutes daily, up to 3 times a week, 2 times a week and sometimes weekly.

The total number of treatments depends on the state of the individual case and it varies from patient to

patient.

iii) <u>Third phase of treatment:</u>

As indicated above, at the end of the treatment course, it is recommended to decrease the amplitude, frequency and time of treatment gradually in the last few treatments. The Ri-2000S may also be used advantageously to complete a treatment course, as the DEMAGNETIZATION phase provided by this system automatically causes the amplitude of the field to decrease at a desired rate, as described hereinbefore.

2. SELECTING "PULSE" FREQUENCY

In general, the <u>FREQUENCY</u> of any periodic process is the number of full cycles within a time interval (second).

<u>Definition of "PULSE" FREQUENCY</u>

For the pulsating system, <u>"pulse" frequency</u> is the repetition frequency of pulse bundles, in bundles per second (Hz). The <u>basic frequency</u> is the frequency of repetition of pulses in each pulse bundle (60 Hz or 50 Hz, depending on the frequency of the sinusoidal current supplied to the control unit);

For the sinusoidal system, <u>"pulse frequency"</u> is the repetition frequency of the sinusoidal wave bundles, in bundles per second (Hz). The <u>basic frequency</u> is the frequency of the sinusoidal field in each sine wave bundle (60 Hz or 50 Hz, depending on the frequency of the sinusoidal current supplied to the control unit).

With the RHUMART-therapy system, a "pulse frequency" range between 1.56 Hz to 60 Hz is used. This frequency range (1.56 - 60 Hz) is divided in two frequency domains which are used for most therapeutic applications: i.e. .....

The <u>LOW FREQUENCY</u> range (below 7.5 Hz) is used for treatment of <u>ACUTE INFLAMMATORY</u> process, and the <u>HIGHER FREQUENCY</u> range (above 25 Hz) is used for treatment of <u>CHRONIC DEGENERATIVE</u> processes.

3. <u>USE OF HIGH DOSAGE</u>

Higher dosage: (i.e. frequency above 25 Hz, intensity of field above 50 Gauss and time period of treatment longer than 10 minutes) should be applied for treating <u>degenerative disorders</u> caused by <u>chronic inflammatory processes</u> such as:

- Osteoporosis;

- Arthrosis of the knee and hip joints, paralyses, fractures, muscular atrophy;

- Osteochondrosis and spondylarthrosis of the spine with degenerative changes in the discs.

4. <u>USE OF LOW DOSAGE</u>

In many <u>acute or painful diseases</u>, low dosages should be used: (frequency below 7.5 Hz, intensity of field below 30 Gauss and time period of treatment below 10 minutes).

5. <u>RHUMART-THERAPY COMBINED TO OTHER TYPES OF MEDICAL PROCEDURES:</u>

RHUMART-therapy can be combined with classical medical procedures such as balneological procedures and chemotherapy (with the exception of antibiotics and bacteriological treatments where RHUMART-therapy is not recommended).

The therapies of nature-cure practitioners such as neural therapy (using electric currents) and ozone therapy (or negative ion therapy); as well as all homeopathic treatment modalities can be advantageously

- 45 -

combined with RHUMART-therapy.

For all the above possibilities of combined procedures, RHUMART-therapy can be used either simultaneously, before or immediately after the treatment combined with RHUMART-therapy.

However, during the entire RHUMART-therapy treatment
course, no diagnostic X-ray nor ionizing radiation
treatments should be carried out.

The embodiments of the invention in which an exclusive property or privilege is claimed are defined as follows:

1.      An electro-magnetic low-frequency therapeutic system comprising a magnetization coil for creating an electro-magnetic field, means to secure said coil in a predetermined fixed position, generator means for feeding said coil with predetermined treatment signals to obtain a desired magnetic field characteristic, control circuit means for selecting desired characteristics of said treatment signals, said control circuit means having (i) circuit control means for controlling the peak intensity of said desired magnetic field, (ii) frequency control means to select the frequency of said treatment signals to obtain a selected type of a plurality of treatment signals, (iii) adjustment means to preset the duration time of said treatment signals and said electro-magnetic field, said desired magnetic field characteristics being predetermined from a magnetic field pattern chart representative of the parameters of the field of said magnetization coil in the surrounding environment of said coil whereby to obtain a desired range of intensity of said field and a desired orientation thereof relative to a position of said coil.

2.      A system as claimed in claim 1 wherein said generator means is a sinusoidal signal generator and wherein said circuit control means further comprises demagnetization control means to preselect a demagnetization time of said electro-magnetic field.

3.      A system as claimed in claim 2 wherein said circuit control means further comprises demagnetization mode selection means to preselect the mode of attenuation of said electro-magnetic field.

4.        A system as claimed in claim 3 wherein said demagnetization mode is selectable between a linear or quasi-exponential demagnetization characteristic.

5.        A system as claimed in claim 1 wherein said generator means is a pulse generator including a pulse shaping circuit comprising essentially two alternating switches for charging and discharging a capacitor, said capacitor discharging through a damping resistance $R_L$ including the resistance of said coil, and a specific coil inductance to produce a desired current pulse shape having a critical damping defined by the following equations:

$$C = \frac{4L}{R_L^2} \quad ; \quad t_m = \frac{2L}{R_L} \quad ; \text{ and } i_m = -\left(\frac{R_L}{2L}\right) \frac{C \, V_0}{e}$$

where C = capacitance of capacitor being discharged into the treatment coil through a damping resistor $R_{Damping}$

   L = inductance of the treatment coil;

   $R_L$ = total series resistance in the discharging circuit of the capacitor; $R_L$ is equal to the sum of the damping resistor $R_{Damping}$ and the D.C. electrical resistance $r_L$ of the treatment coil winding;

   $t_m$ = time lapse between beginning of the current pulse and the time corresponding to the maximum current $i_m$, as illustrated in FIG. 8 herein;

   $t_i$ = period of time from the beginning of the current pulse to the time where the second derivative of the current in this pulse is equal to zero (we have shown that $t_i = t_m/2$); (see FIG. 8 herein);

$i_m$ = maximum current flowing through the treatment coil;

$V_0$ = initial voltage across the capacitor C, before discharging it into the treatment coil;

$e$ = 2.7182818..., being the limit approached by the expression $(1 + \frac{1}{n})^n$ as n approaches infinity, also called the "base" of natural or neperian logarithm.

6.      A system as claimed in claim 5, wherein said control means for controlling the intensity of said magnetic field is an adjustable auto-transformer provided in a high voltage circuit which includes said pulse shaping circuit, said transformer having a winding fed by an A.C. current supply and having a tap connection to said charging capacitor connected to said coil to produce said current pulse of selected intensity, said pulse being generated at the frequency of said A.C. supply and having a repetition rate being controlled by a frequency circuit.

7.      A system as claimed in claim 6 wherein said frequency circuit comprises said frequency control means to provide a predetermined delay between said discharge pulses in accordance with a desired value preset by an adjustable control member whereby to obtain a series of said pulses on a continuous or periodically interrupted basis.

8.      A system as claimed in claim 7 wherein there is further provided a detector circuit to detect the zero signal crossings of said alternating charging circuit, said detector circuit synchronizing said frequency circuit.

9. A system as claimed in claim 4 wherein during said attenuation of said electromagnetic field the peak intensity of said field is automatically varied in accordance with said selected mode by means of an amplitude circuit feeding a mode circuit, and a motor control circuit controlled by said mode circuit and an initiating circuit, said motor control circuit controlling a variable auto-transformer provided in a high voltage circuit.

10. A system as claimed in claim 9 wherein said high voltage circuit includes said circuit control means to adjust the peak amplitude of the current fed by said auto-transformer by controlling an adjustable tap setting of a second auto-transformer connected to an A.C. supply, said auto-transformer being connected to said coil to produce a current signal therein.

11. A system as claimed in claim 10 wherein said high intensity current signal is interrupted in accordance to the desired interruption rate of said magnetic field by means of a switch controlled by a frequency circuit.

12. A system as claimed in claim 11 wherein said frequency circuit comprises said frequency control means to provide said desired interruption rate of said current signal in accordance with a value preset by an adjustable control member.

13. A system as claimed in claim 10 wherein said motor control circuit operates an adjustable tap on said first auto-transformer to demagnetize said coil in accordance with a selected one of said demagnetization modes.

14.    A system as claimed in claim 1 wherein there is further provided switch means to initiate said electro-magnetic field and timer circuits associated with said control circuit means, and indicator means to indicate the presence of said electromagnetic field.

15.    A system as claimed in claim 14 wherein said switch means is connected to an initiating circuit, said initiating circuit controlling the operation of an audible tone circuit, said indicator means being a visual indicator lamp connected in a current monitor circuit.

16.    A system as claimed in claim 1 wherein said field pattern chart comprises displays of the orientation of isomagnetic field lines of said coil to define the pattern of the intensity, and displays of the direction of said magnetic field in space, said field lines being relative to a scale with respect to said coil.

17.    A system as claimed in claim 1 wherein said magnetization coil is one of two hollow cylinder coils having a different desired maximum field intensity, or a core coil having a further desired maximum field intensity, said coils being interchangeable and having substantially the same inductance.

18.    A system as claimed in claim 1 wherein said control means for controlling the intensity is an adjustable control element positionable at ten different positions to vary said intensity in increments of 10% per positions.

19.    A system as claimed in claim 1 wherein said frequency control means is an adjustable control element selectively positionable to provide a desired interruption of a desired one of a plurality of treatment signals of the magnetic field.

20.    A system as claimed in claim 1 wherein said adjustment means to preset the duration time includes an adjustable control element to select a desired time lapse for feeding said coil with said treatment signals.

21.    A system as claimed in claim 20 wherein said adjustment means further includes an adjustable control element to select a desired demagnetization time period of said desired time lapse for demagnetizing said coil in a selected one of a linear or quasi-exponential mode.

22.    A system as claimed in claim 1 wherein said coil is mounted on an articulatable arm, an articulated joint member interconnecting said coil and said arm, said joint member comprising a holding plate securable to said coil, a holding rod secured to said plate and supporting a holding cylinder member, a fastener member in threaded engagement with an end of said rod protruding through said cylinder member, said cylinder member having a retention bore to secure an end of said arm therein.

23.    A system as claimed in claim 22 wherein said retention bore is a hole extending transverse to an arcuate side wall of said cylinder member, a slot in said cylinder member extending in a portion of said side wall across said bore, securing means applying pressure across said slot to compress wall sections of said bore when tightened to frictionally secure said end of said arm in said bore.

24.    A system as claimed in claim 23 wherein a friction disc is provided about said holding rod intermediate said holding plate and an adjacent end face of said cylinder member, said fastener member adjusting the orientation of the longitudinal axis of said arm and fixing its position stationary when in threaded engagement against an opposed end face of said cylinder member to apply clamping pressure.

25.    A system as claimed in claim 1 wherein said coil is a core coil, said coil being maintained in said fixed position in a U-shaped box-like support, said coil having a treatment end which is adjustable in height by adjustable support means insertable under said treatment end, and a cushion member secured forwardly of said box relative to said treatment end.

26.    A method of obtaining a desired electromagnetic field at a chosen location in space in a close surrounding environment of a magnetization coil comprising the steps of:

    i)    providing a field pattern chart of said coil to display the orientation of iso-magnetic field lines of said coil to define the pattern of the intensity, and to display the direction of said magnetic field in space, said field lines being relative to a scale with respect to said coil;

ii) selecting an area in said field pattern chart having a desired field intensity range and field directions;

iii) orienting said coil with respect to a desired location in space to position said selected area thereon;

iv) generating controlled treatment signals to said coil to obtain said desired field intensity range and to select a time/frequency/ amplitude/modulation of said field intensity; and

v) applying said treatment signals for generating said magnetic field for a predetermined period of time.

27. A method as claimed in claim 26 wherein said step (iv) comprises

a) selecting the peak intensity of said desired magnetic field by adjustable control means; and

b) selecting the interruption frequency of said treatment signals by further adjustable control means.

28. A method as claimed in claim 27 wherein said treatment signals are sinusoidal signals, said step (iv) further comprising the steps of

a) selecting a demagnetization time of said desired magnetic field by further adjustable control means, and

b) selecting a demagnetization mode from a linear or quasi-exponential characteristic.

29.     A method as claimed in claim 27 wherein said step (iv) comprises generating treatment pulses by charging and discharging a capacitor to produce discharge pulses having a critical or nearly critical damping characteristic, and controlling the interruption rate of said discharge pulses by a frequency circuit controlled by step (b).

30.     A method as claimed in claim 29 wherein said step of charging said capacitor comprises (a) feeding an auto-transformer with an A.C. signal voltage, (b) feeding the voltage at a tap on said winding of said transformer to said capacitor and (c) activating alternating switches to charge and discharge said capacitor.

31.     A method as claimed in claim 30 wherein said step (c) comprises detecting the zero signal crossings of said A.C. signal voltage, and controlling said switches in accordance with a selected one of said interruption rate.

32.     A method as claimed in claim 26 wherein there is further provided the step of indicating the presence of said treatment signals generating said magnetic field.

33.    An electro-magnetic low frequency therapeutic
system comprising a magnetization coil for creating an
electro-magnetic field in response to a preselected
treatment signal whereby said magnetic field has a desired
treatment characteristic, said magnetic field having an
adjustable intensity set by adjustable control means, said
control means also having (i) circuit means for controlling the peak amplitude of the field for achieving a
specific modulation of said peak amplitude in a given
time, (ii) means to select the orientation and/or direction with respect to a tissue to be treated, (iii) circuit
means to select the repetition frequency, either pulsating
or sinusoidal, fed to the magnetization coil to obtain a
selected one of different therapeutic signals, (iv) means
to select a desired treatment time; automatic demagnetizing means responsive to a preselected demagnetizing time
and mode for attenuating said field in a desired manner
to terminate a magnetic field treatment.

FIG. 1

FIG. 2

65    20,39, OR 56    57    60

(23,24, ETC...)    64    63    58    59

67    66    61    62

(21,22,37,38 OR 46)

(A) DISK BRAKE DETAILS

47(a)    47

48    56

56

47(b)    48

(B)

48

49

50

51

(C)

FIG. 3

CONTROL UNIT OR GENERATOR — TREATMENT UNIT OR TREATMENT APPLICATOR — MECHANICAL SUPPORT

(A) PULSATING RHUMART SYSTEM Ri-2000 P

(B) SINUSOIDAL RHUMART SYSTEM Ri-2000 S

0048451

3/16

FIG.4

FIG. 5

FIG. 6

# FIG. 7

Ri-2000 RHUMART™-THERAPY SYSTEM

POWER · MAGNETIC FIELD · TREATMENT

FREQUENCY(Hz) (60Hz supply) (50Hz supply) · MAG TIME(MIN) · DEMAG TIME(MIN)

ON

AMPLITUDE · MODE · LINEAR / EXP

RODROL INSTRUMENTATION INC

9/16

0048451

8/16

0048451

## FIG. 8

(A)

$R_L$ = DAMPING RESISTANCE: (including that of coil)

L = INDUCTANCE OF COIL: .MINI (core coil) .MOYI .MAXI } (air coils)

(B)  $t_i = 2t_m$

(C)  $t_i = 2t_m$

FIG. 9

FIG. 10

FIG. 11: HIGH VOLTAGE CIRCUIT (SINUSOIDAL OPTION) 111S

FIG 12 (PULSE)

112 AC SUPPLY

113 DC VOLTAGE REGULATOR

TREATMENT COIL — 11, 12, OR 13

102 AMPLITUDE

HIGH VOLTAGE CIRCUIT — 111

OP1   OP3 OP4 OP2

114

109 CURRENT MONITOR CIRCUIT

103 FREQUENCY

FREQUENCY CIRCUIT — 116

2C

108 AUDIBLE TONE CIRCUIT

115 INITIATION CIRCUIT — Q, R

107 START

117 DELAY CIRCUIT — DL

104 MAG TIME

FIG 13: HIGH VOLTAGE CIRCUIT (PULSATING OPTION) — 111 P

N.B.: ALL CAPACITORS IN µF, AND ALL RESISTORS IN Ω.

FIG. 14    (SINUSOIDAL)

FIG. 15

FIG. 16 (PULSATING)

## DOCUMENTS CONSIDERED TO BE RELEVANT

| Category | Citation of document with indication, where appropriate, of relevant passages | Relevant to claim |
|---|---|---|
| X | FR - A - 2 290 224 (ELMATRON)<br><br>* claims 1,2; page 3, lines 6-15 *<br><br>--- | 1,12,<br>17-20,<br>22,23,<br>26,27,<br>33 |
|  | FR - A - 2 448 354 (BEAULIEU)<br><br>* page 3, lines 30-35; page 4, lines 10-23; page 2, line 35 to page 3, line 1 *<br><br>& CA - A - 1 102 752<br><br>--- | 1-3,<br>6,14,<br>17,20,<br>25,26,<br>33 |
| A | DE - A - 2 553 197 (BUSCHKY)<br><br>* page 4 *<br><br>--- | 1,6,<br>12,18-<br>20,26,<br>27,33 |
|  | DE - A - 2 707 574 (GÖDDE)<br><br>* page 14, paragraph 2; page 24; page 28, the last three lines; page 29, first line; page 31, paragraph 3 *<br><br>--- | 1,3,<br>22,26,<br>33 |
|  | FR - A - 2 169 327 (E.S.B.)<br><br>* page 12, line 16 to page 13, line 7; page 10, line 26 to page 11, line 20; page 25, line 28; figure 3 *<br><br>& CA - A - 987 391 | 1,5,<br>12,19,<br>25,26,<br>33 |
|  | MEDICAL AND BIOLOGICAL ENGINEERING, vol. 11, no. 1, January 1973, STEVENAGE (GB)<br>P.A. ÖBERG: "Magnetic stimulation of nerve tissue", pages 55-63<br><br>* page 58, the two last paragraphs; page 61, figure 11; page 62, left-hand column, ./. | 2-5 |

**CLASSIFICATION OF THE APPLICATION (Int. Cl.³)**

A 61 N 1/42

**TECHNICAL FIELDS SEARCHED (Int. Cl.³)**

A 61 N 1/42
A 61 N 1/40

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 10.12.1981 | SIMON |

EPO Form 1503.1   06.78

0048451

| | European Patent Office | **EUROPEAN SEARCH REPORT** | Application number EP 81 10 7360 |
|---|---|---|---|

- 2 -

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | paragraph 4 * | | |
| D | US - A - 3 841 305 (HALLGREN) <br><br> * column 6, line 33 to column 7, line 20; column 7, lines 39-45 * | 5 | |
| | FR - A - 674 332 (BOULITTE) <br><br> * page 1, lines 34-49 * | 5 | |
| E | EP - A - 0 039 206 (INOUE) <br><br> * page 19, line 23 to page 20, line 18; page 22, line 4 to page 23, line 12; figure 9 * | 1,2,5, 12,18, 19,25, 26,33 | TECHNICAL FIELDS SEARCHED (Int. Cl.³) |

EPO Form 1503.2  06.78